# EUROPÄISCHE PATENTSCHRIFT

(11) **EP 0 635 996 B1**
(45) Veröffentlichungstag und Bekanntmachung des Hinweises auf die Patenterteilung: **11.02.1998**
(21) Anmeldenummer: 90917518.4
(22) Anmeldetag: 26.11.1990
(51) Int. Cl.: A01N 25/32, A01N 43/56, C07D 231/06, C07D 231/54

(54) **PYRAZOLINE ZUM SCHUTZ VON KULTURPFLANZEN GEGENÜBER HERBIZIDEN**
PYRAZOLINES FOR THE PROTECTION OF CROP PLANTS AGAINST HERBICIDES
PYRAZOLINE POUR LA PROTECTION DE PLANTES CULTIVEES CONTRE LES HERBICIDES

(30) Priorität: 30.11.1989 DE 3939503
(43) Veröffentlichungstag der Anmeldung: 01.02.1995
(73) Patentinhaber: Hoechst Schering AgrEvo GmbH, 13342 Berlin (DE)
(72) Erfinder: RÖSCH, Wolfgang, D-6000 Frankfurt am Main (DE); SOHN, Erich, D-8900 Augsburg (DE); BAUER, Klaus, D-6450 Hanau (DE); BIERINGER, Hermann, D-6239 Eppstein (DE)
(86) Internationale Anmeldenummer: EP9002020
(87) Internationale Veröffentlichungsnummer: WO9107874

(56) Entgegenhaltungen:
- EP-A- 0 174 562
- EP-A- 0 268 554
- EP-A- 0 269 806
- WO-A-88/06583
- DE-A- 3 808 896

## Beschreibung

Bei der Anwendung von Herbiziden, können unerwünschte, nicht tolerierbare Schäden an Kulturpflanzen auftreten. Besonders bei der Applikation von Herbiziden nach dem Auflaufen der Kulturpflanzen besteht daher oft das Bedürfnis, das Risiko einer möglichen Phytotoxizität zu vermeiden.

Solche Verbindungen, die die Eigenschaften besitzen, Kulturpflanzen gegen phytotoxische Schäden durch Herbizide zu schützen, ohne die eigentliche herbizide Wirkung dieser Mittel zu beeinträchtigen, werden "Antidot" oder "Safener" genannt.

Verschiedene Verbindungen wurden für diese Anwendung bereits beschrieben (vgl. z.B. EP-A 152 006 und EP-A 0174562).

In der Deutschen Patentanmeldung P-3 808 896.7 wurden 1-Phenyl- und 1-(Pyrid-2-yl)-pyrazolderivate als Safener vorgeschlagen.

Die Anwendung von Alkoxypyrazolinen als Safener wurde in der Deutschen Patentanmeldung P 3923649.8 (HOE 89/F 235) vorgeschlagen.

Gegenstand der vorliegenden Patentansprüche sind kulturpflanzenschützende Mittel, welche 4,5-Pyrazolin-3-carbonsäureesterderivate der Formel (I), 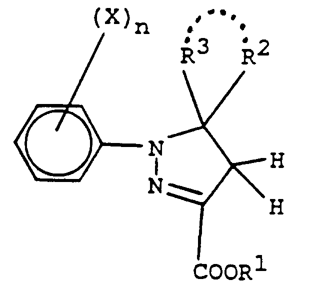 worin
- X: unabhängig voneinander Halogen oder Halogenalkyl,
- n: eine ganze Zahl von 1 bis 3,
- R¹: Wasserstoff, Alkyl, Cycloalkyl, Trialkylsilyl, Trialkylsilylmethyl oder Alkyloxyalkyl,
- R² und R³: unabhängig voneinander Wasserstoff, Alkyl, C₃-C₆-Cycloalkyl, Alkenyl, Alkinyl, Halogenalkyl, Alkoxyalkyl, Hydroxyalkyl, Alkoxycarbonyl, Alkylcarbonyl, Alkylaminocarbonyl, gegebenenfalls substituiertes Phenyl, Halogen oder Cyano bedeuten, wobei die Reste R² und R³ mit dem 5-C-Atom des Pyrazolinrings einen Ring bilden können,
enthalten.

Die Formel (I) umfaßt dabei alle möglichen geometrischen Isomeren und Stereoisomeren. In Formel (I) bedeuten Halogen Fluor, Chlor, Brom oder Jod, Alkyl geradkettiges, verzweigtes oder cyclisches Alkyl, Alkenyl geradkettiges oder verzweigtes Alkenyl, wobei die Doppelbindung an beliebiger Stelle im Alkenylrest vorliegen kann und Alkinyl geradkettiges oder verzweigtes Alkinyl, wobei auch hier die Dreifachbindung beliebig im Alkinylrest lokalisiert sein kann, Halogenalkyl ein- oder mehrfach durch Halogen substituiertes Alkyl, Alkoxyalkyl und Hydroxyalkyl ein- oder mehrfach durch Alkoxy bzw. Hydroxy substituiertes Alkyl. Die genannten Bedeutungen für Alkyl gelten auch für die in Kombinationen wie Alkyloxyalkyl, Alkyloxycarbonyl und Alkylaminocarbonyl enthaltenen Alkylreste.

Von besonderem Interesse sind erfindungsgemäße Mittel mit Verbindungen der Formel (I), worin
- X: unabhängig voneinander Halogen oder C₁-C₄-Halogenalkyl,
- n: ein ganze Zahl von 1 bis 3,
- R¹: C₁-C₆-Alkyl, C₃-C₆-Cycloalkyl, Tri-(C₁-C₄-alkyl)-silyl, Tri-(C₁-C₄-alkyl)silylmethyl oder (C₁-C₆-Alkyloxy)-C₁-C₆-alkyl,
- R² und R³: unabhängig voneinander Wasserstoff, C₁-C₆-Alkyl, C₂-C₆-Alkenyl, C₂-C₆-Alkinyl, C₃-C₆-Cycloalkyl, C₁-C₆-Halogenalkyl, Mono- oder Di-(C₁-C₄-Alkoxy)-C₁-C₄-Alkyl, C₁-C₆-Hydroxyalkyl, (C₁-C₆-Alkyl)carbonyl, Mono- oder Di-(C₁-C₄-alkyl)amino-carbonyl, Cyano, Halogen, (C₁-C₁₂-Alkyl)-oxycarbonyl, Phenyl oder Phenyl, das durch ein oder mehrere Reste aus der Gruppe Halogen, C₁-C₄-Alkyl, C₁-C₄-Alkoxy oder Cyano substituiert ist, bedeuten.

Halogenalkyl bedeutet bevorzugt Trifluormethyl, 2-Chlorethyl, 1,1,2,2-Tetrafluorethyl oder Hexafluorpropyl; Halogen ist bevorzugt Fluor, Chlor oder Brom.
Alkyl steht bevorzugt für einen der Reste Methyl, Ethyl, n-Propyl, i-Propyl, die Butyl-, Pentyl und Hexylisomeren, Cyclopentyl und Cyclohexyl.
Alkenyl bedeutet bevorzugt einen der Reste Vinyl, 1-Propen-1-yl, 1-Propen-2-yl, die Butenyl-, Pentenyl- und Hexenylisomeren.
Alkinyl steht bevorzugt für Ethinyl, 1-Propinyl oder 2-Propinyl.

Bevorzugt sind erfindungsgemäße Mittel mit Verbindungen der Formel (I), worin
- X: unabhängig voneinander Fluor, Chlor, Brom oder Trifluormethyl, vorzugsweise jeweils in 2- oder 4-Position,
- n: 2 oder 3,
- R¹: C₁-C₄-Alkyl oder (C₁-C₄-Alkoxy)-C₁-C₄-alkyl,
- R²: Wasserstoff, C₁-C₄-Alkyl, C₂-C₄-Alkenyl,
- R³: C₁-C₄-Alkyl, C₃-C₄-Alkenyl, C₂-C₄-Alkinyl, C₁-C₄-Halogenalkyl, Mono- oder Di-(C₁-C₄-Alkoxy)-C₁-C₄-alkyl, C₁-C₄-Hydroxyalkyl, Mono- oder Di-(C₁-C₄-alkyl)-aminocarbonyl, Cyano, (C₁-C₁₂-Alkyloxy)-carbonyl, Phenyl oder Phenyl, das ein- oder mehrfach durch Halogen, insbesondere Fluor oder Chlor, substituiert ist,
bedeuten.

Besonders bevorzugt sind erfindungsgemäße Mittel mit Verbindungen der Formel (I), worin
- X: unabhängig voneinander Fluor, Chlor, Brom oder Trifluormethyl, vorzugsweise in 2- oder 4-Position im Phenylring,
- n: 2 oder 3,
- R²: Wasserstoff, C₁-C₄-Alkyl, C₂-C₄-Alkenyl oder C₂-C₄-Alkinyl,
- R³: C₁-C₄-Alkyl, das unsubstituiert oder ein- oder mehrfach durch Halogen substituiert ist, (C₁-C₁₂-Alkyl)oxycarbonyl oder Cyano
bedeuten.

Die Verbindungen der Formel (I) sind zum Teil aus WO 88/06583 als Vorprodukte zur Herstellung von Insektiziden bekannt und können analog den dort beschriebenen Verfahren hergestellt werden. Die Safenerwirkung der Verbindungen der Formel (I) ist nicht bekannt gewesen.

Gegenstand der Erfindung sind auch nicht vorbeschriebene Verbindungen der genannten Formel (I), in der
- R¹: Cycloalkyl, bevorzugt C₃-C₆-Cycloalkyl, Trialkylsilyl, Trialkylsilylmethyl oder Alkyloxyalkyl, bevorzugt (C₁-C₆-Alkoxy)-C₁-C₆-alkyl bedeutet oder Verbindungen der Formel (I), in der
- (X)ₙ: 2 oder 3 Reste am Phenylring, vorzugsweise zwei Reste in 2,3- oder 2,4-Position, insbesondere in 2,4-Position am Phenylring, aus der Gruppe Halogen und Halogenalkyl, wie z.B C₁-C₄-Halogenalkyl, vorzugsweise die Reste 2,4-Cl₂, 2,4-F₂, 2,4-Br₂, 2-Cl-4-F, 2-F-4-Cl, 2,4-(CF₃)₂, 2-CF₃-4-Cl, 2-Cl-4-CF₃, 2-F-4-CF₃, 2-CF₃-4-F, 2-CF₃-4-Br, 2-Br-4-CF₃, insbesondere 2,4-Cl₂
bedeuten.

Die obengenannten Verbindungen der Formel (I) können beispielsweise hergestellt werden, indem man eine Verbindung der Formel (II), 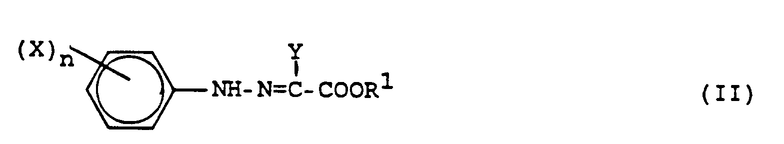 worin Y für Chlor oder Brom steht und (X)ₙ und R¹ die oben angegebenen Bedeutungen haben, mit Olefinen der Formel (III), 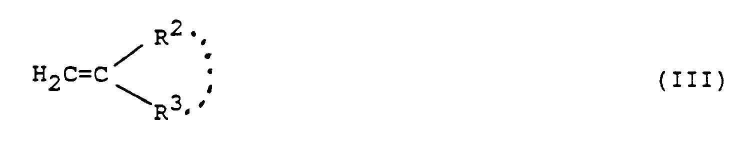 worin R² und R³ die oben angegebenen Bedeutungen haben, umsetzt.

Die Komponenten können äquimolar oder im Überschuß der Verbindungen der Formel (III) eingesetzt werden, zweckmäßigerweise im molaren Verhältnis von 1 : 1,05 bis 1 : 20, bevorzugt im molaren Verhältnis 1 : 1,1 bis 1 : 5.

Die Verbindungen der Formel (II) sind zum Teil bekannt oder können nach üblichen Verfahren synthetisiert werden. Sie lassen sich beispielsweise aus den entsprechenden Anillnen durch Diazotieren und Kuppeln mit den entsprechenden 2-Chlor-Acetessigestern erhalten. Die Verbindungen der Formel (III) sind ebenfalls nach üblichen Verfahren zugänglich, zum Beispiel durch Wittig-Olefinierung der entsprechenden Ketone oder Aldehyde der Formel R²COR³.

Die Umsetzung der Verbindungen der Formeln( II) und (III) wird in der Regel zwischen 0 und 150°C, vorteilhaft zwischen 20 und 100°C, gegebenenfalls in Gegenwart einer organischen Base, wie sterisch gehinderte Amine, z.B. Triethylamin oder Pyridin, oder einer anorganischen Base, wie z.B. Kaliumcarbonat, Kaliumhydroxyd oder Natriumcarbonat, mit oder ohne Gegenwart eines organischen Lösungsmittels, wie gegebenenfalls eines halogenierten aliphatischen oder aromatischen Kohlenwasserstoffs oder eines Ethers, beispielsweise des Lösungsmittels Toluol, Xylol, Dichlorethan, Dimethoxyethan, Di- oder Triglyme, Cyclohexan, Petrolether oder Chlorbenzol, durchgeführt.

Basen und Lösungsmittel sind nur beispielhaft aufgezählt, ohne daß das Verfahren auf diese Beispiele beschränkt ist.

Die Verbindungen der Formel (I) haben die Eigenschaft, phytotoxische Nebenwirkungen von Herbiziden, beim Einsatz in Nutzpflanzenkulturen zu vermindern oder ganz zu verhindern. Die Verbindungen der Formel (I) sind in der Lage, schädliche Nebenwirkungen der Herbizide weitgehend oder völlig aufzuheben, ohne die Wirksamkeit dieser Herbizide gegen Schadpflanzen zu schmälern. Das Einsatzgebiet herkömmlicher Herbizide kann durch Zugabe der Safenerverbindung der Formel (I) ganz erheblich vergrößert werden.

Gegenstand der vorliegenden Erfindung ist daher auch ein Verfahren zum Schutz von Kulturpflanzen gegen phytotoxische Nebenwirkungen von Herbiziden, das dadurch gekennzeichnet ist, daß man die Pflanzen, Pflanzensamen oder Anbauflächen mit einer Verbindung der Formel (I) vor, nach oder gleichzeitig mit einem Herbizid behandelt.

Herbizide, deren phytotoxische Nebenwirkungen mittels der Verbindungen der Formel (I) herabgesetzt werden können, sind z.B. Carbamate, Thiocarbamate, Halogenacetanilide, substituierte Phenoxy-, Naphthoxy- und Phenoxyphenoxycarbonsäurederivate sowie Heteroaryloxyphenoxycarbonsäurederivate wie Chinolyloxy-, Chinoxalyloxy-, Pyridyloxy-, Benzoxazolyloxy-, Benzthiazolyloxy-phenoxy-carbonsäureester und ferner Cyclohexandion-derivate. Bevorzugt hiervon sind Phenoxyphenoxy und Heteroaryloxyphenoxy-carbonsäureester und strukturelle Analoga wie Benzylphenoxycarbonsäureester. Als Ester kommen hierbei insbesondere niedere Alkyl-, Alkenyl- und Alkinylester in Frage.

Beispielsweise seien, ohne daß dadurch eine Beschränkung erfolgen soll, folgende Herbizide genannt:
A) Herbizide vom Typ der Phenoxyphenoxy- und Heteroaryloxyphenoxycarbonsäure-(C₁-C₄)-alkyl, (C₂-C₄)-alkenyl- oder (C₃-C₄)-alkinylester wie 2-(4-(2,4-Dichlorphenoxy)-phenoxy)-propionsäuremethylester, 2-(4-(4-Brom-2-chlorphenoxy)-phenoxy)-propionsäuremethylester, 2-(4-(4-Trifluormethylphenoxy)-phenoxy)-propionsäuremethylester, 2-(4-(2-Chlor-4-trifluormethylphenoxy)-phenoxy)-propionsäuremethylester; 2-(4-(2,4-Dichlorbenzyl)-phenoxy)-propionsäuremethylester, 2-Isopropylideneaminooxyethyl(R)-2-[4-(6-chloroquinoxalin-2-yloxy)-phenoxy]-propionat (Propaquizafop), 4-(4-(4-Trifluormethylphenoxy)-phenoxy)-pent-2-ensäureethylester, 2-(4-(3,5-Dichlorpyridyl-2-oxy)-phenoxy)-propionsäureeethylester, 2-(4-(3,5-Dichlorpyridyl-2-oxy)-phenoxy)-propionsäurepropargylester, 2-(4-(6-Chlorbenzoxazol-2-yloxy)-phenoxy)-propionsäureethylester, 2-(4-(6-Chlorbenzthiazol-2-yl-oxy)-phenoxy)-propionsäureethylester, 2-(4-(3-Chlor-5-trifluormethyl-2-pyridyloxy)-phenoxy)-propionsäuremethylester, 2-(4-(5-Trifluormethyl-2-pyridyloxy)-phenoxy)-propionsäurebutylester, 2-(4-(6-Chlor-2-chinoxalyloxy)-phenoxy)-propionsäureethylester, 2-(4-(6-Fluor-2-chinoxalyloxy)-phenoxy)-propionsäureethylester, 2-(4-(5-Chlor-3-fluorpyridyl-2-oxy)-phenoxy)-propionsäurepropargylester, 2-(4-(6-Chlor-2-chinolyloxy)-phenoxy)-propionsäureethylester, 2-(4-(3,5-Dichlorpyridyl-2-oxy)-phenoxy)-propionsäuretrimethylsilylmethylester, 2-(4-(3-Chlor-5-trifluormethoxy-2-pyridyloxy)-phenoxy)- propionsäureethylester,
B) Chloracetanilid-Herbizide wie N-Methoxymethyl-2,6-diethyl-chloracetanilid, 2-Chlor-N-(2-ethyl-6-methylphenyl)-N-(2-methoxy-1-methylethyl)-acetamid, N-(3-Methyl-1,2,4-oxdiazol-5-yl-methyl)-chloressigsäure-2,6-dimethylanilid,
C) Cyclohexandion-derivate wie S-Ethyl-N,N-dipropylthiocarbamat oder S-Ethyl-N,N-diisobutylthiocarbamat,
D) Cyclohexadion-Derivate wie 2-(N-Ethoxybutyrimidoyl)-5-(2-ethylthiopropyl)-3-hydroxy-2-cyclohexen-1-on, 2-(N-Ethoxybutyrimidoyl)-5-(2-phenylthiopropyl)-3-hydroxy-2-cyclohexen-1-on oder 2-(1-Allyloxyiminbutyl)-4-methoxycarbonyl-5,5-dimethyl-3-oxocyclohexenol, 2-(N-Ethoxypropionamidoyl)-5-mesityl-3-hydroxy-2-cyclohexen-1-on (oder auch als 5-(2,4,6-Trimethylphenyl)-3-hydroxy-2-[1-(Ethoxyimino)-propyl]-cyclohex-2-en-1-on bezeichnet), 2-(N-Ethoxybutyrimidoyl)-3-hydroxy-5-(thian-3-yl)-2-cyclohexen-1-on, 2-[1-(Ethoxyimino)-butyl]-3-hydroxy-5-(2H-tetrahydrothiopyran-3-yl)-2-cyclohexen-1-one (BASF 517); (±)-2-[(E)-3-chloroallyloxyiminopropyl]-5-(2-ethylthiopropyl)-3-hydroxycyclohex-2-enone (Clethodim).

Von den Herbiziden, welche erfindungsgemäß mit den Verbindungen der Formel (I) kombiniert werden können, sind bevorzugt die unter A) aufgeführten Verbindungen zu nennen, insbesondere 2-(4-(6-Chlorbenzoxazol-2-yl-oxy)-phenoxy)-propionsäureethylester, 2-(4-(6-Chlorbenzthiazol-2-yl-oxy)-phenoxy)-propionsäureethylester und 2-(4-(5-Chlor-3-fluorpyridyl-2-oxy)-phenoxy)-propionsäurepropargylester. Von den unter D) genannten Substanzen ist insbesondere 2-(N-Ethoxypropionamidoyl)-5-mesityl-3-hydroxy-2-cyclohexen-1-on von Bedeutung.

Das Gewichtsverhältnis Safener (Verbindung I) : Herbizid kann innerhalb weiter Grenzen variieren und ist vorzugsweise im Bereich von 1 : 10 bis 10 : 1, insbesondere 2 : 1 bis 1 : 10.

Die jeweils optimalen Mengen an Herbizid und Safener sind abhängig vom Typ des verwendeten Herbizids oder vom verwendeten Safener sowie von der Art des zu behandelnden Pflanzenbestandes und lassen sich von Fall zu Fall durch entsprechende Versuche ermitteln.

Haupteinsatzgebiete für die Anwendung der Safener sind vor allem Getreidekulturen (Weizen, Roggen, Gerste, Hafer), Reis, Mais, Sorghum, aber auch Baumwolle, Zuckerrüben, Zuckerrohr und Sojabohne.

Die Safener können je nach ihren Eigenschaften zur Vorbehandlung des Saatgutes der Kulturpflanze (Beizung der Samen) verwendet werden oder vor der Saat in die Saatfurchen eingebracht werden und vor, nach oder gleichzeitig zusammen mit dem Herbizid vor oder nach dem Auflaufen der Pflanzen angewendet werden. Vorauflaufbehandlung schließt sowohl die Behandlung der Anbaufläche vor der Aussaat als auch die Behandlung der angesäten, aber noch nicht bewachsenen Anbauflächen ein.

Bevorzugt ist jedoch die gleichzeitige Anwendung des Safeners mit dem Herbizid in Form von Tankmischungen oder Fertigformulierungen.

Die Verbindungen der Formel (I) oder deren Kombination mit einem oder mehreren der genannten Herbizide bzw. Herbizidgruppen können auf verschiedene Art formuliert werden, je nachdem wie es durch die biologischen und/oder chemisch-physikalischen Parameter vorgegeben ist. Als Formulierungsmöglichkeiten kommen beispielsweise folgende infrage:

Emulgierbare Konzentrate (EC), Emulsionen (EW), Suspensionskonzentrate (SC), Kapselsuspension (CS), wasserlösliche Konzentrate (SL), wasserlösliche Pulver (SP), wasserlösliche Granulate (SG), wasserdispergierbare Pulver (Spritzpulver) (WP), wasserdispergierbare Granulate (WG), ölmischbare Lösungen (OL), Stäubemittel (DP), Granulate (GR) in Form von Mikro-, Sprüh-, Aufzugs- und Adsorptionsgranulaten, ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag, München, 4. Auflage 1986; van Valkenburg, "Pesticides Formulations", Marcel Dekker N.Y., 2nd Ed. 1972-73; K. Martens, "Spray Drying Handbook", 3rd Ed. 1979, G. Goodwin Ltd, London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldewell N.J.; H. v. Olphen, "Introduction to Clay Colloid Chemistry", 2nd Ed., J. Wiley & Sons, N.Y. 1950; McCutcheon's "Detergents and Emulsifiers Annual", MC Publ. Corp., Ridgewood N.J.; Sisley and Wood, "Encyclopedia of Suface Active Agents", Chem. Publ. Co. Inc., N.Y. 1964; Schönfeldt, "Grenzflächenaktive Äthylenoxiaddukte", Wiss. Verlagsgesellschaft, Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix. Spritzpulver sind in Wasser gleichmäßig dispergierbare Präparate, die neben dem Wirkstoff außer einem Verdünnungs- oder Inertstoff noch Netzmittel, z.B. polyoxethylierte Alkyphenole, polyoxethylierte Fettalkohole und Fettamine, Fettalkoholpolyglykolethersulfate, Alkan- oder Alkylbenzolsulfonate und Dispergiermittel, z.B. ligninsulfonsaures Natrium, 2,2'-dinaphthylmethan-6,6'-disulfonsaures Natrium, dibutylnaphthalin-sulfonsaures Natrium oder auch oleylmethyltaurinsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitanfettsäureester oder Polyoxethylensorbitester.

Stäubemittel erhält man durch Vermahlen des Wirkstoffes mit fein verteilten festen Stoffen, z.B. Talkum, natürlichen Tonen wie Kaolin, Bentonit und Pyrophyllit, oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In der Regel enthalten die erfindungsgemäßen Formulierungen 1 bis 95 Gew.-%, vorzugsweise 2 bis 90 Gew.-% Wirkstoff, d.h. Wirkstoff der Formel (I) oder Kombination des Wirkstoffs der Formel (I) mit dem Pflanzenschutzmittel (Herbizid).

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 85 Gew.-%, vorzugsweise 5 bis 80 Gew.-% betragen. Staubförmige Formulierungen enthalten meistens 1 bis 25 Gew.-%, vorzugsweise 5 bis 20 Gew.-% an Wirkstoff, versprühbare Lösungen etwa 0,2 bis 25 Gew.-%, vorzugsweise 2 bis 20 Gew.-% Wirkstoff. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids u. a. variiert die erforderliche Aufwandmenge der Verbindungen der Formel (I). Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Folgende Beispiele dienen zur Erläuterung der Erfindung:

### A. Formulierungsbeispiele

a) Ein Stäubemittel wird erhalten, indem man 10 Gew.-Teile einer Verbindung der Formel (I) und 90 Gew.-Teile Talkum oder Inertstoff mischt und in einer Schlagmühle zerkleinert.
b) Ein in Wasser leicht dispergierbares, benetzbares Pulver wird erhalten, indem man 25 Gew.-Teile einer Verbindung der Formel (I), 64 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) mit 6 Gew.-Teilen Alkylphenolpolyglykolether (®Triton X 207), 3 Gew.-Teilen Isotridecanolpolyglykolether (8 EO = 8 Ethylenoxyeinheiten) und 71 Gew.-Teilen paraffinischem Mineralöl (Siedebereich z.B. ca. 255 bis über 277°C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.
e) Ein in Wasser leicht emulgierbares Konzentrat aus einem Phenoxycarbonsäureester und einem Antidot (10 : 1) wird erhalten aus
   - 12,00 Gew.-%: 2-[4-(6-Chlorbenzoxazol-2-yl-oxy)-phenoxy]-propionsäureethylester,
   - 1,20 Gew.-%: Verbindung der Formel (I),
   - 69,00 Gew.-%: Xylol,
   - 7,80 Gew.-%: dodecylbenzolsulfonsaurem Calcium,
   - 6,00 Gew.-%: ethoxyliertem Nonylphenol (10 EO) und
   - 4,00 Gew.-%: ethoxyliertem Rizinusöl (40 EO).

   Die Zubereitung erfolgt wie unter Beispiel a) angegeben.
f) Ein in Wasser leicht emulgierbares Konzentrat aus einem Phenoxycarbonsäureester und einem Antidot (1 : 10) wird erhalten aus
   - 4,0 Gew.-%: 2-[4-(6-Chlorbenzoxazol-2-yl-oxy)-phenoxy]-propionsäureethylester,
   - 40,0 Gew.-%: Verbindung der Formel (I),
   - 30,0 Gew.-%: Xylol,
   - 20,0 Gew.-%: Cyclohexanon,
   - 4,0 Gew.-%: dodecylsulfonsaurem Calcium und
   - 2,0 Gew.-%: ethoxyliertem Rizinusöl (40 EO).
g) Ein in Wasser dispergierbares Granulat wird erhalten, indem man
   - 75 Gewichtsteile: einer Verbindung der Formel (I),
   - 10 Gewichtsteile: ligninsulfonsaures Calcium,
   - 5 Gewichtsteile: Natriumlaurylsulfat,
   - 3 Gewichtsteile: Polyvinylalkohol und
   - 7 Gewichtsteile: Kaolin
   mischt, auf einer Stiftmühle mahlt und das Pulver in einem Wirbelbett durch Aufsprühen von Wasser als Granulierflüssigkeit granuliert.
h) Ein in Wasser dispergierbares Granulat wird auch erhalten, indem man
   - 25 Gewichtsteile: einer Verbindung der Formel (I),
   - 5 Gewichtsteile: 2,2'-dinaphthylmethan-6,6'-disulfonsaures Natrium,
   - 2 Gewichtsteile: oleoylmethyltaurinsaures Natrium,
   - 1 Gewichtsteile: Polyvinylalkohol,
   - 17 Gewichtsteile: Calciumcarbonat und
   - 50 Gewichtsteile: Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.
i) Ein nach üblichen Methoden hergestelltes Granulat besteht z.B. aus
   - 2 - 15 Gew.-Teilen: Wirkstoff der Formel (I) und
   - 98 - 85 Gew.-Teilen: inertes Granulatmaterial, wie Attapulgit, Bimsstein und Quarzsand.

### Beispiel 1:

### B. Herstellungsbeispiele

### 1-(2,4-Dichlorphenyl)-5-methyl-5-dodecyloxycarbonylpyrazolin-3-carbonsäureethylester

31,8 g Methylacrylsäuredodecylester und 37,6 g Triethylamin werden auf 70°C erwärmt. Innerhalb einer halben Stunde läßt man zu dieser Mischung 14,8 g des 2,4-Dichlorphenylhydrazons von 2-Chlorglyoxalsäureethylester, Formel (II) mit X¹=X²=Y=Cl, R¹=C₂H₅ (IIa) in 50 ml Toluol zutropfen. Man rührt 4 h bei 80°C nach, saugt nach Abkühlen vom Niederschlag ab und engt im Vakuum schonend sein. Nach Säulenchromatographie (Laufmittel n-Heptan/Essigester 1:1) über Kieselgel erhält man 19,0 g des oben bezeichneten Pyrazolins als Öl mit einem Brechungsindex von n_{D}(20°C): 1,5198.

### Beispiel 2:

### 1-(2,3-Dichlorphenyl)-5-cyano-5-methyl-pyrazolin-3-carbonsäuremethylester

19,0 g Methylacrylnitril und 7,6 g Triethylamin werden auf 70°C erwärmt. Innerhalb einer halben Stunde läßt man zu dieser Mischung 14,8 g des 2,3-Dichlorphenylhydrazons von 2-Chlorglyoxalsäureethylester, (IIb), in 50 ml Dimethoxyethan zutropfen. Man rührt 4 h bei 80°C nach, saugt nach Abkühlen vom Niederschlag ab und engt im Vakuum schonend ein. Aus der Mutterlauge fällt ein farbloser Niederschlag (9,2 g) vom Schmelzpunkt 66-67°C aus.

### Beispiel 3:

### 1-(2,4-Dichlorphenyl)-5-methyl-5-ethoxycarbonyl-pyrazolin-3-carbonsäureethylester

22,8 g Methylacrylsäureethylester und 14,8 g Verbindung der Formel (II a) (siehe Beispiel 1), werden auf 50-60°C erwärmt. Innerhalb einer halben Stunde läßt man zu dieser Mischung 7,6 g Triethylamin zutropfen. Man rührt weitere 2 h bei 70°C nach, saugt nach Abkühlen vom Niederschlag ab und engt unter reduziertem Druck schonend ein. Man erhält 18,1 g blaßgelbes Öl;
Brechungsindex: n_{D}(20°C):1,5651.

### Beispiel 4:

### 1-(2,4-Dichlorphenyl)-5-methyl-5-phenyl-pyrazolin-3-carbonsäureethylester

23,7 g 2-Methylstyrol und 14,8 g Verbindung (IIa) (siehe Beispiel 1), werden zusammen mit 50 ml gesättigter wäßriger Natriumcarbonat-Lösung 4 h auf 80°C erhitzt. Anschließend wird die wäßrige Phase abgetrennt, die organische Phase über Natriumsulfat getrocknet und unter reduziertem Druck eingeengt. Nach Säulenchromatographie (Laufmittel n-Heptan/Essigester 1:1) über Kieselgel erhält man 6,9 g des oben bezeichneten Pyrazolins als farblosen Feststoff mit einem Schmelzpunkt von 87-89°C.

In der folgenden Tabelle 1 sind weitere Verbindungen der Formel (I) aufgeführt, die analog den Verfahren der Beispiele 1 bis 4 erhalten werden.

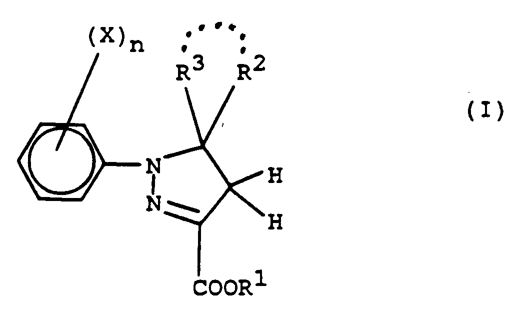

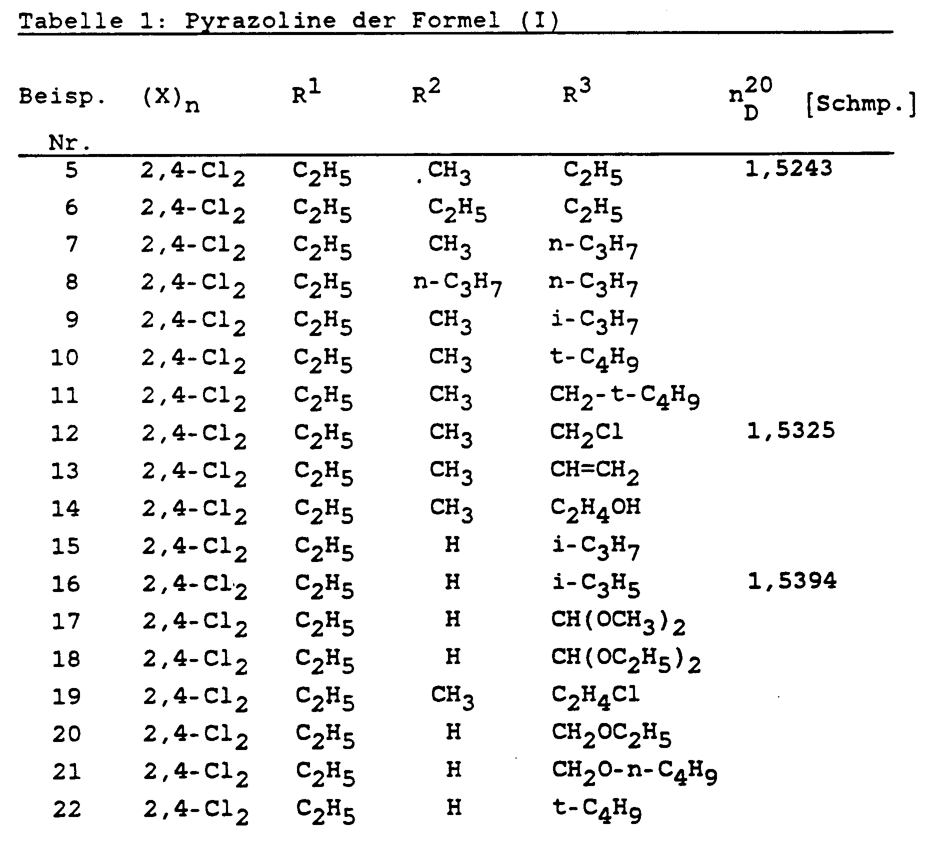

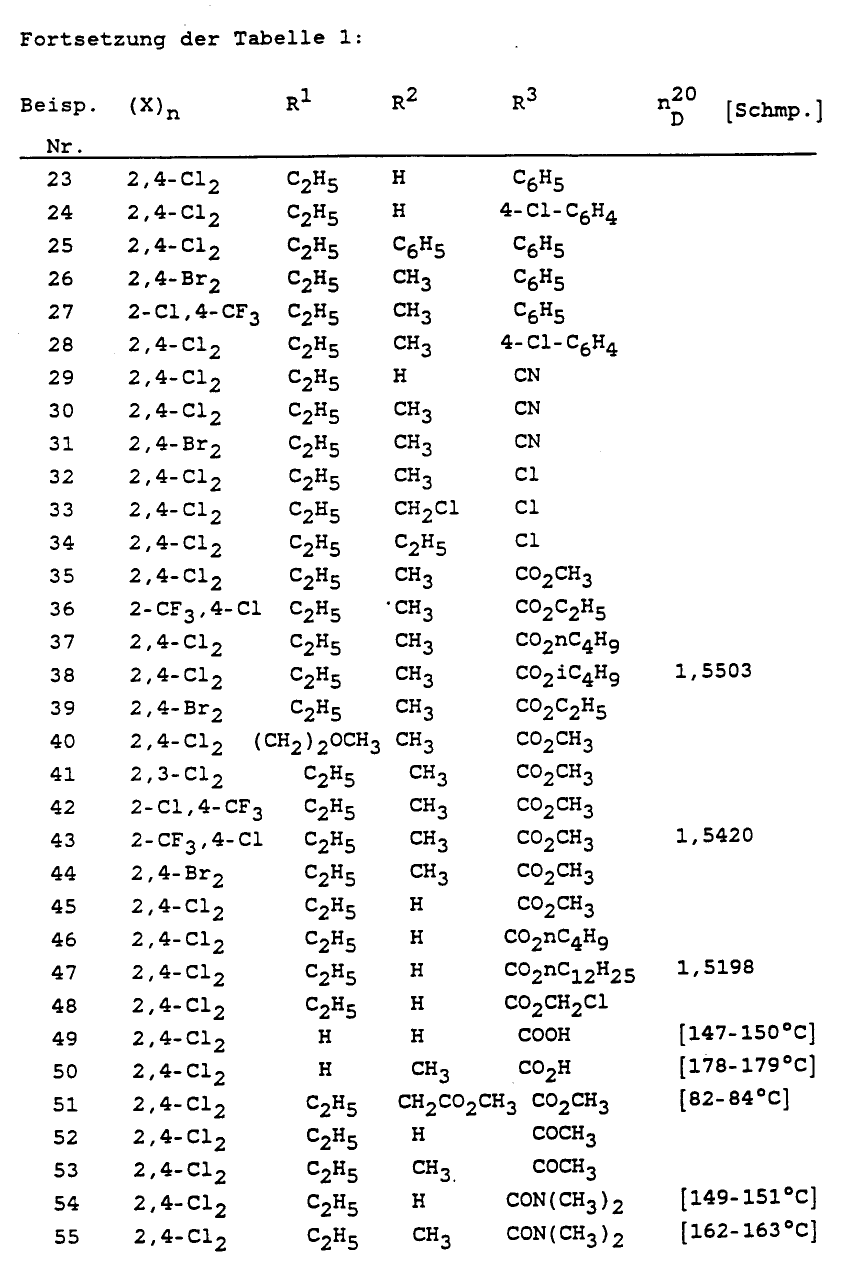

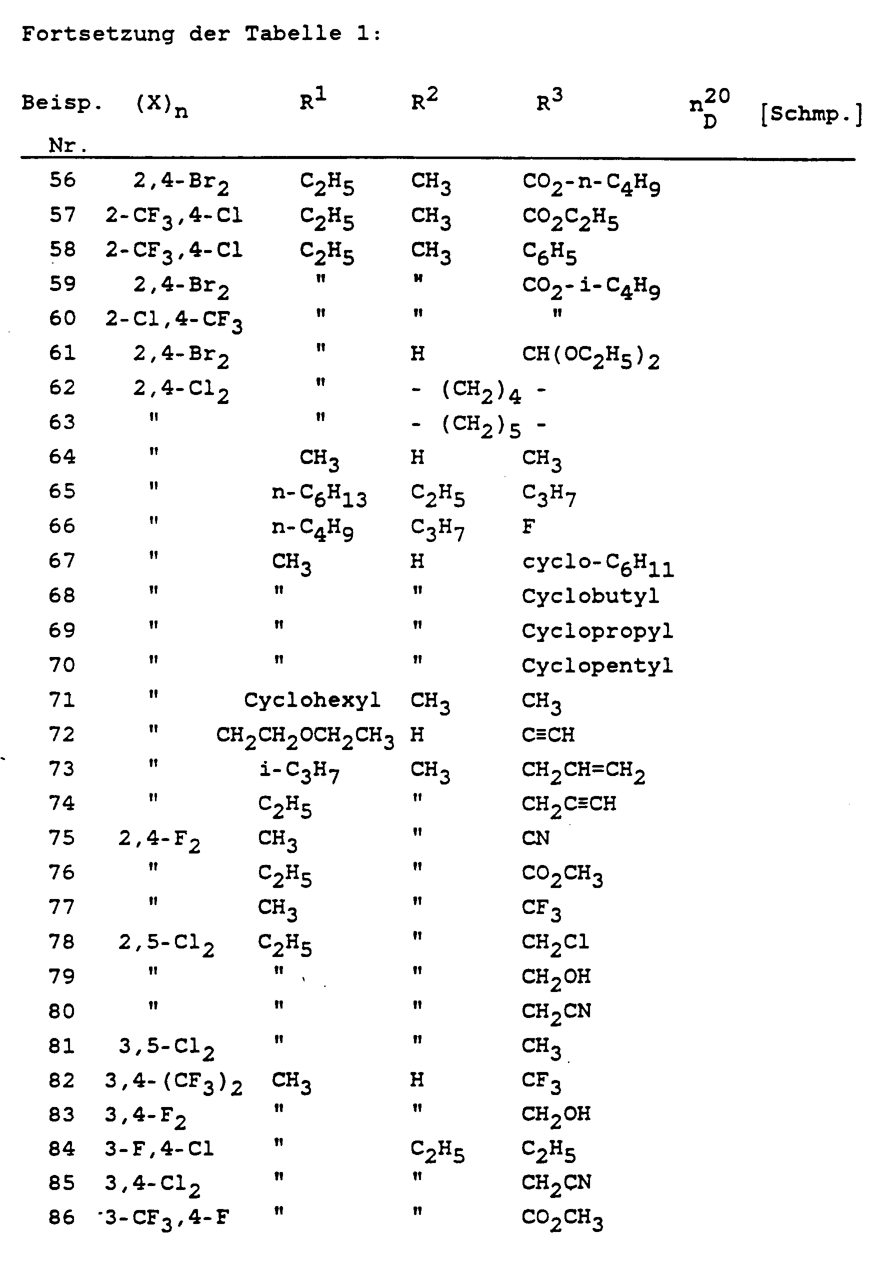

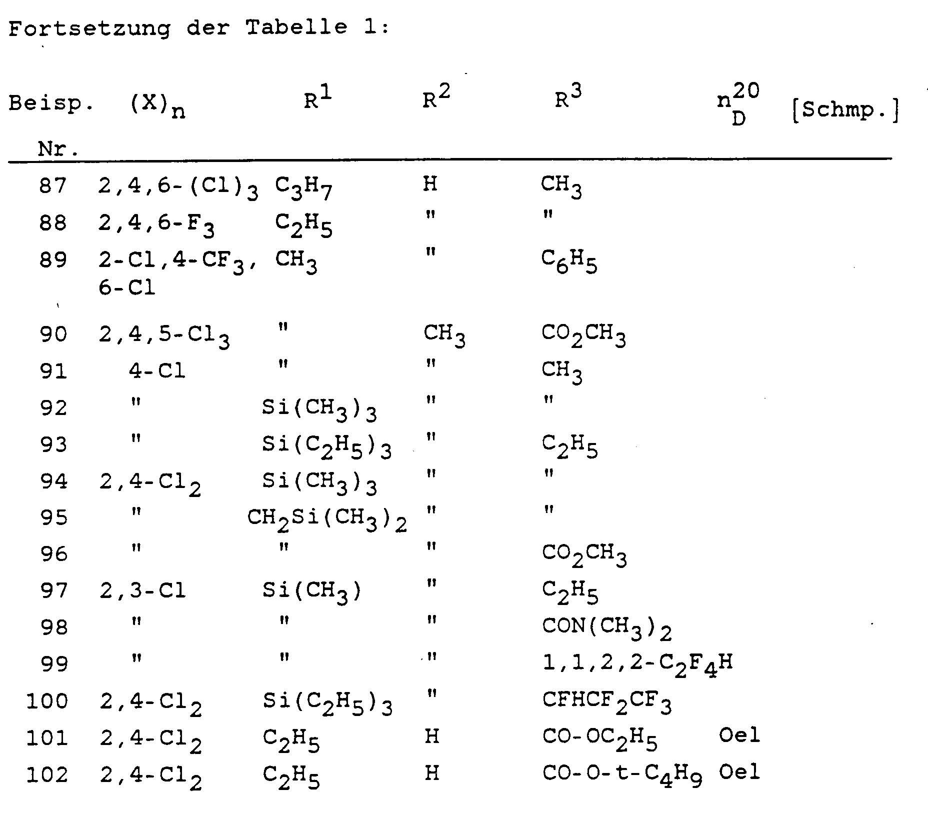

### Beispiel 1

Weizen und Gerste wurden im Gewächshaus in Plastiktöpfen bis zum 3 bis 4-Blattstadium herangezogen und dann mit erfindungsgemäßen Safener-Verbindungen und Herbiziden im Nachauflaufverfahren behandelt. Die Herbizide und die Verbindungen der Formel (I) wurden dabei in Form wäßriger Suspensionen bzw. Emulsionen mit einer Wasseraufwandmenge von umgerechnet 800 l/ha ausgebracht. 3 bis 4 Wochen nach der Behandlung wurden die Pflanzen visuell auf jede Art von Schädigung durch die ausgebrachten Herbizide bonitiert, wobei insbesondere das Ausmaß der anhaltenden Wachstumshemmung berücksichtigt wurde. Der Grad der Schädigung bzw. die Safener-Wirkung von Verbindungen der Formel (I) alleine bzw. in Kombination mit Herbiziden wurde in % Schädigung bestimmt.

Die Ergebnisse zeigen (vgl.Tabelle 2), daß die erfindungsgemäßen Verbindungen starke Herbizidschäden an Kulturpflanzen effektiv reduzieren können.

Selbst bei starken Überdosierungen eines Herbizids wie Fenoxaprop-ethyl werden bei Kulturpflanzen auftretende schwere Schädigungen deutlich reduziert, und geringere Schäden völlig aufgehoben. Mischungen aus Herbiziden und erfindungsgemäßen Verbindungen eignen sich deshalb in vorteilhafter Weise zur selektiven Unkrautbekämpfung in Getreidekulturen.

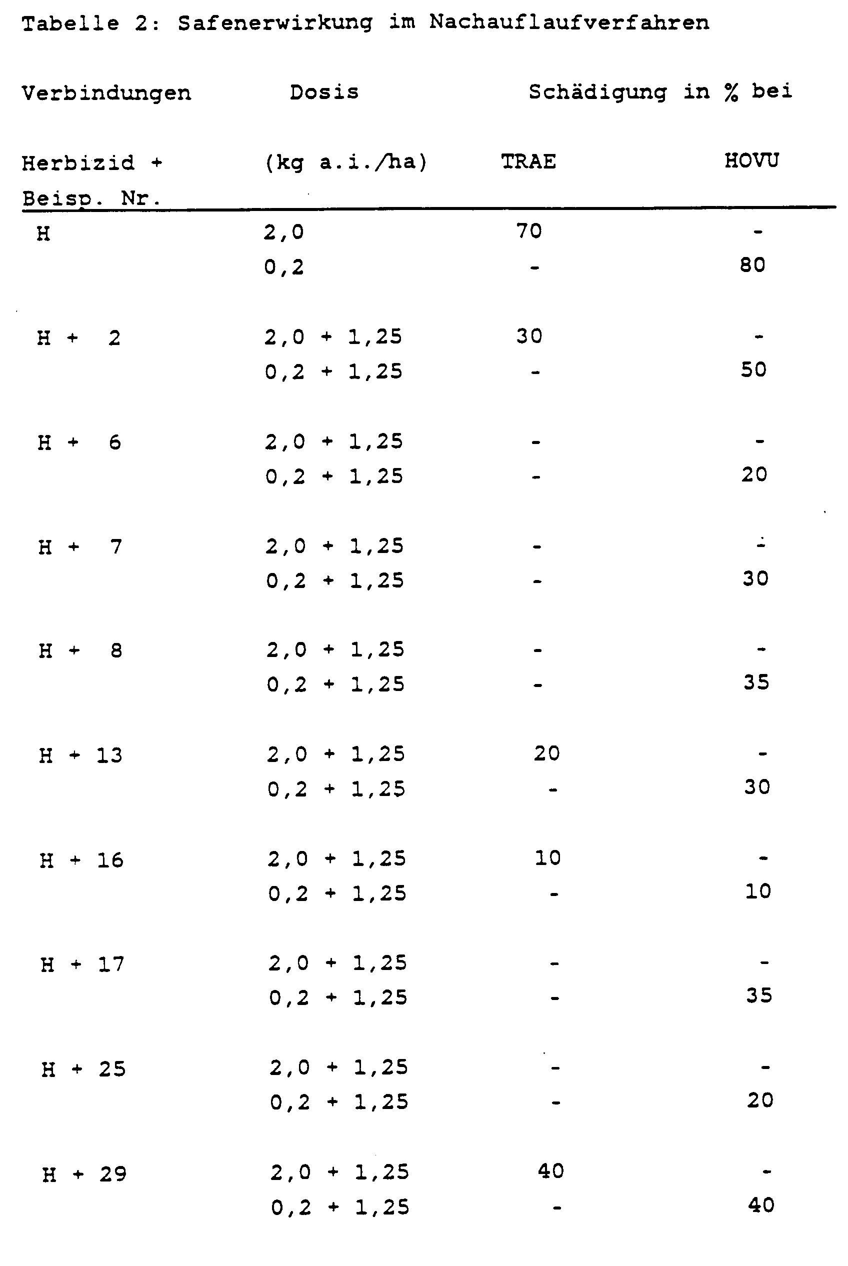

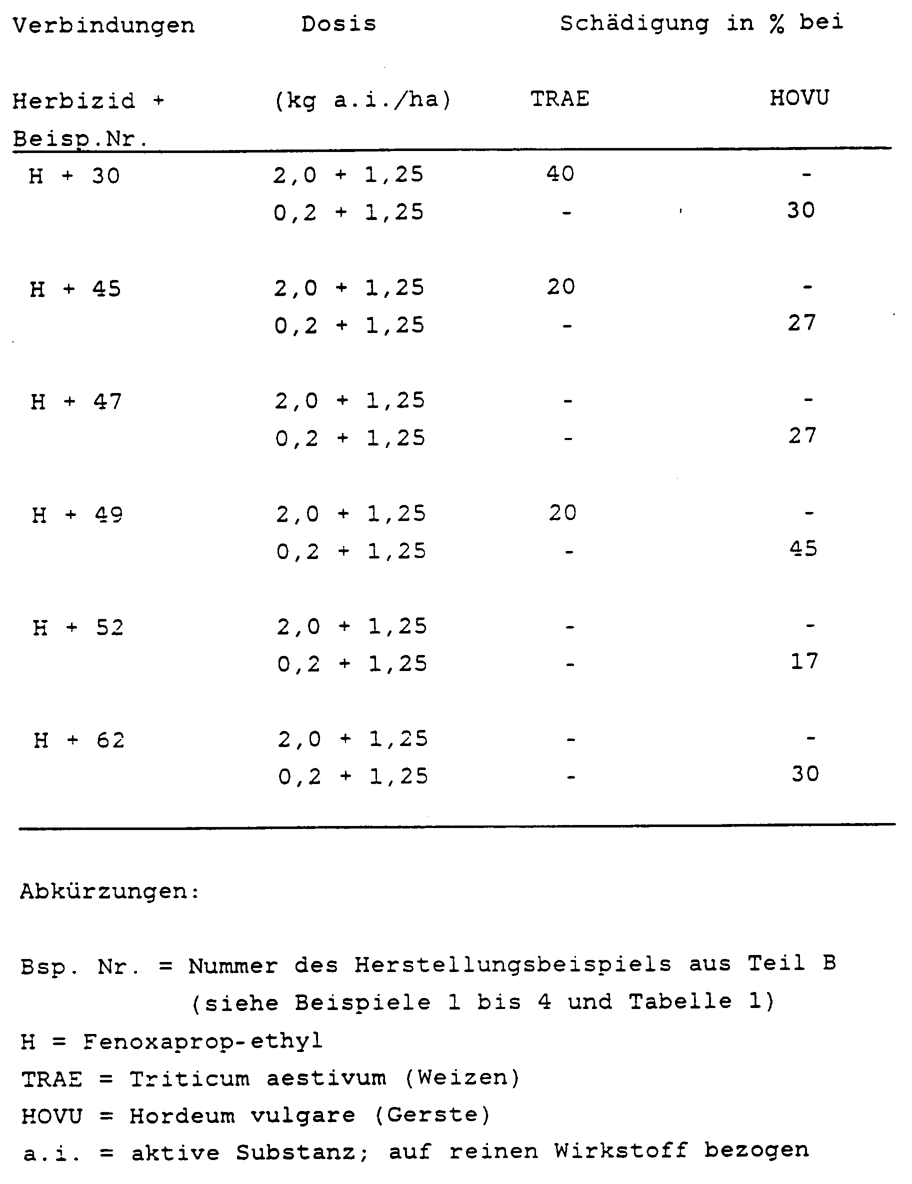

### Beispiel 2

In einer Versuchsserie analog Beispiel 1, die aber mit einer größeren Zahl an Wiederholungsversuchen bei der jeweiligen Applikation durchgeführt wurde, erhielt man die in Tabelle 3 gezeigten Ergebnisse. Teilweise abweichende absolute Werte in den Wirksamkeiten sind durch klimatische Einflüsse erklärbar, die bei beiden Versuchsserien nicht ganz gleich waren.

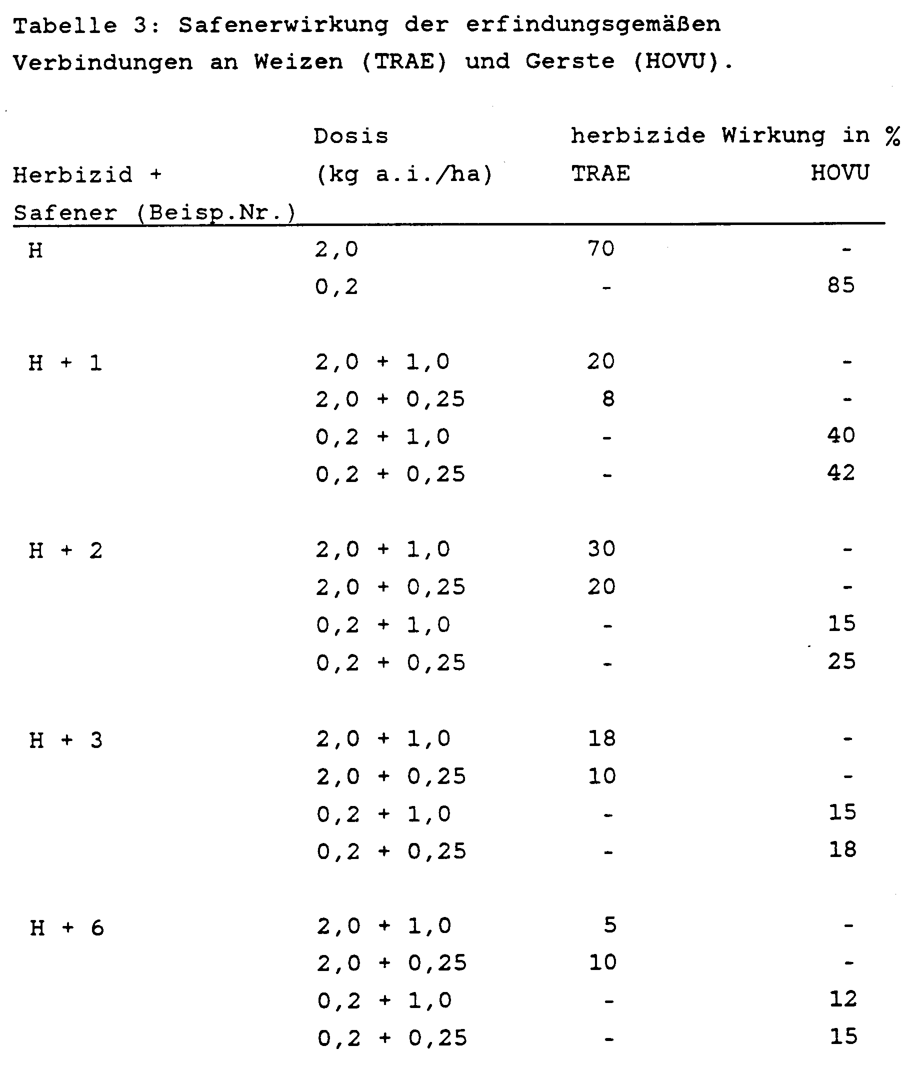

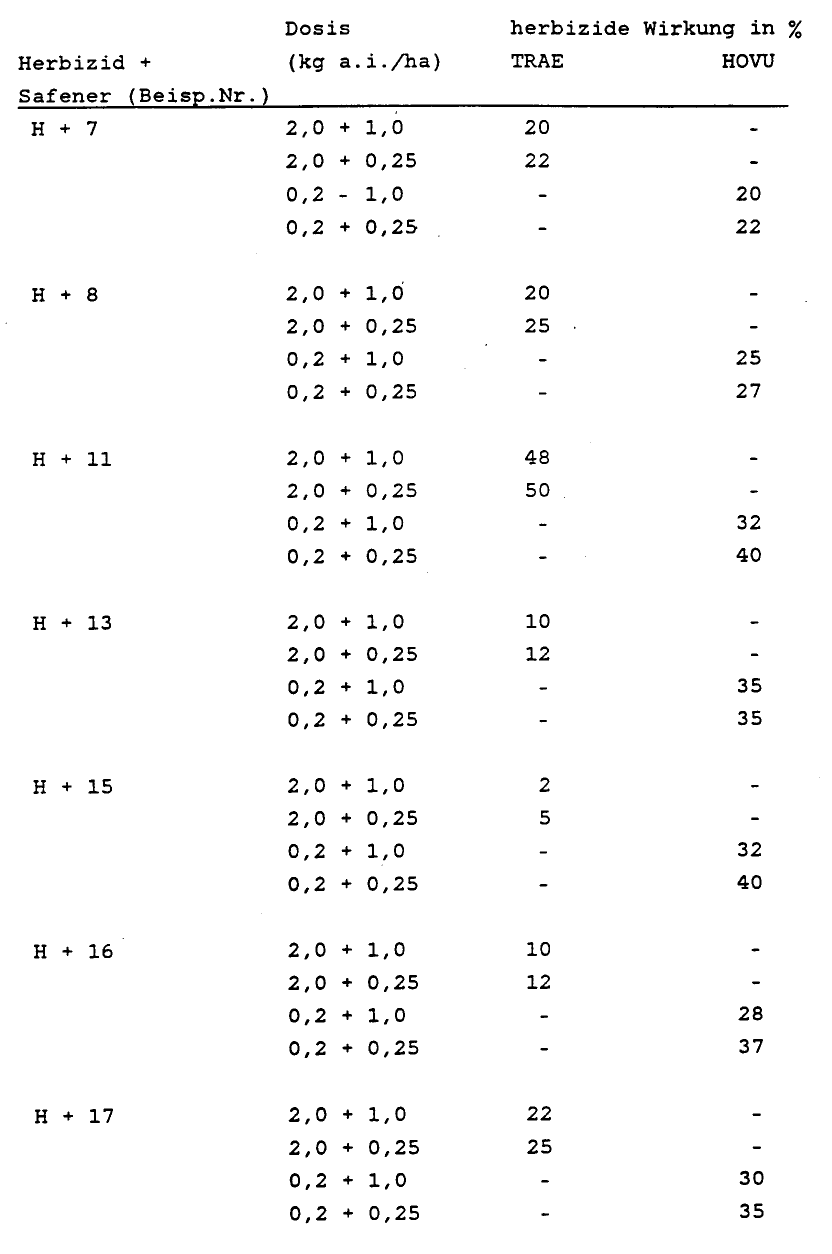

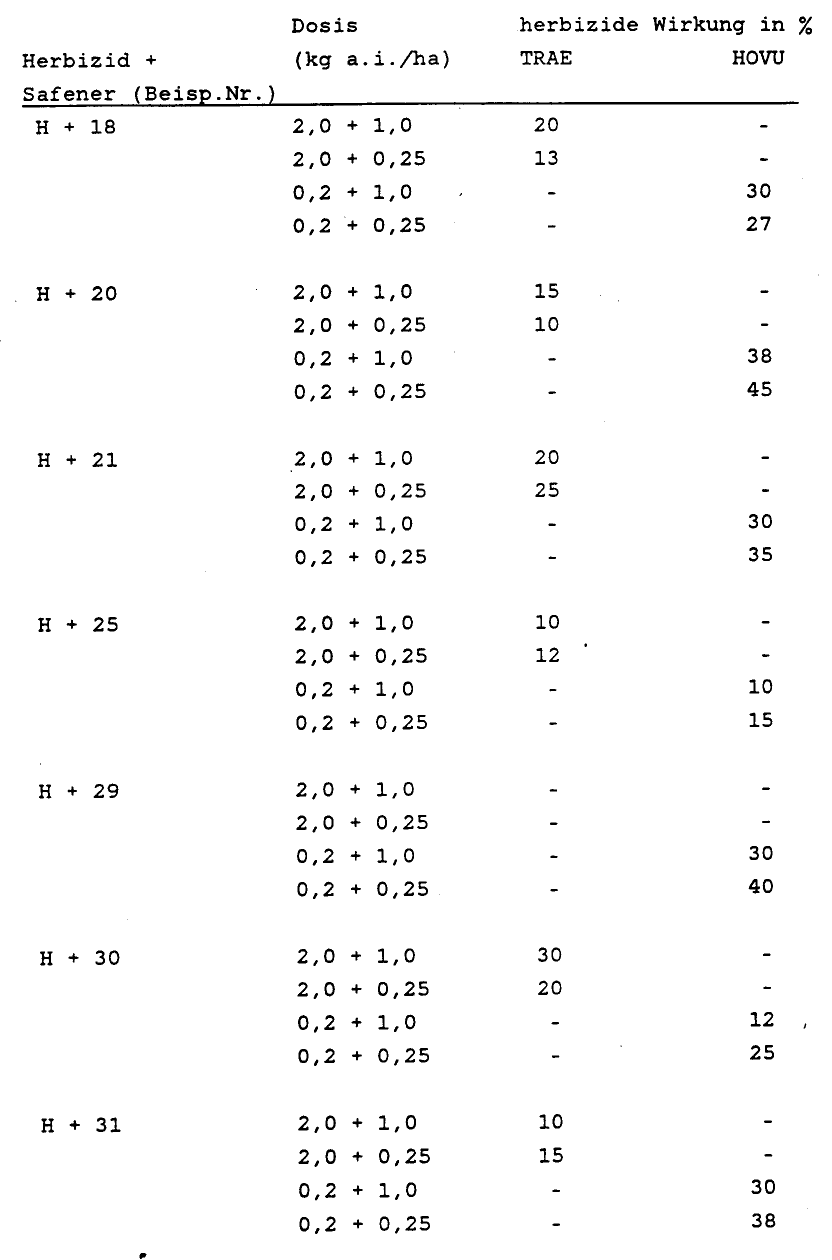

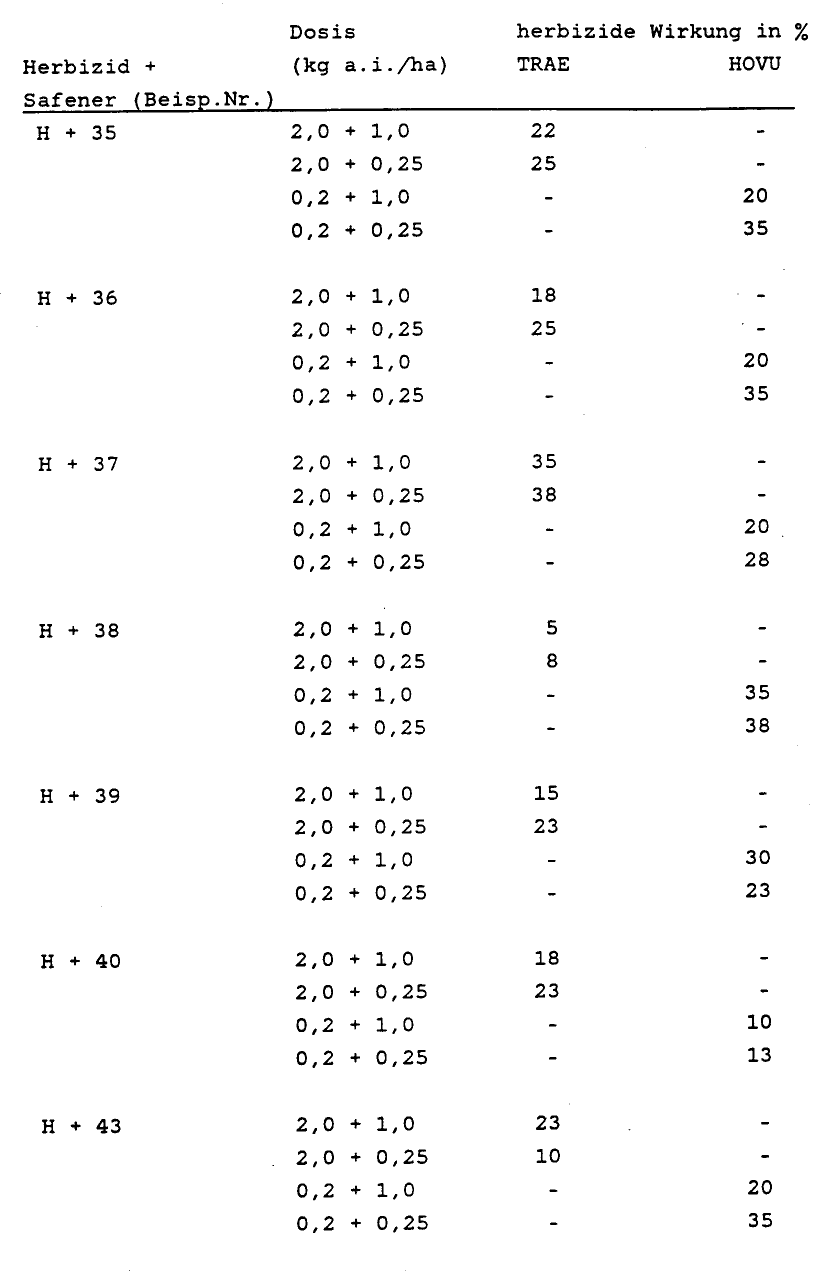

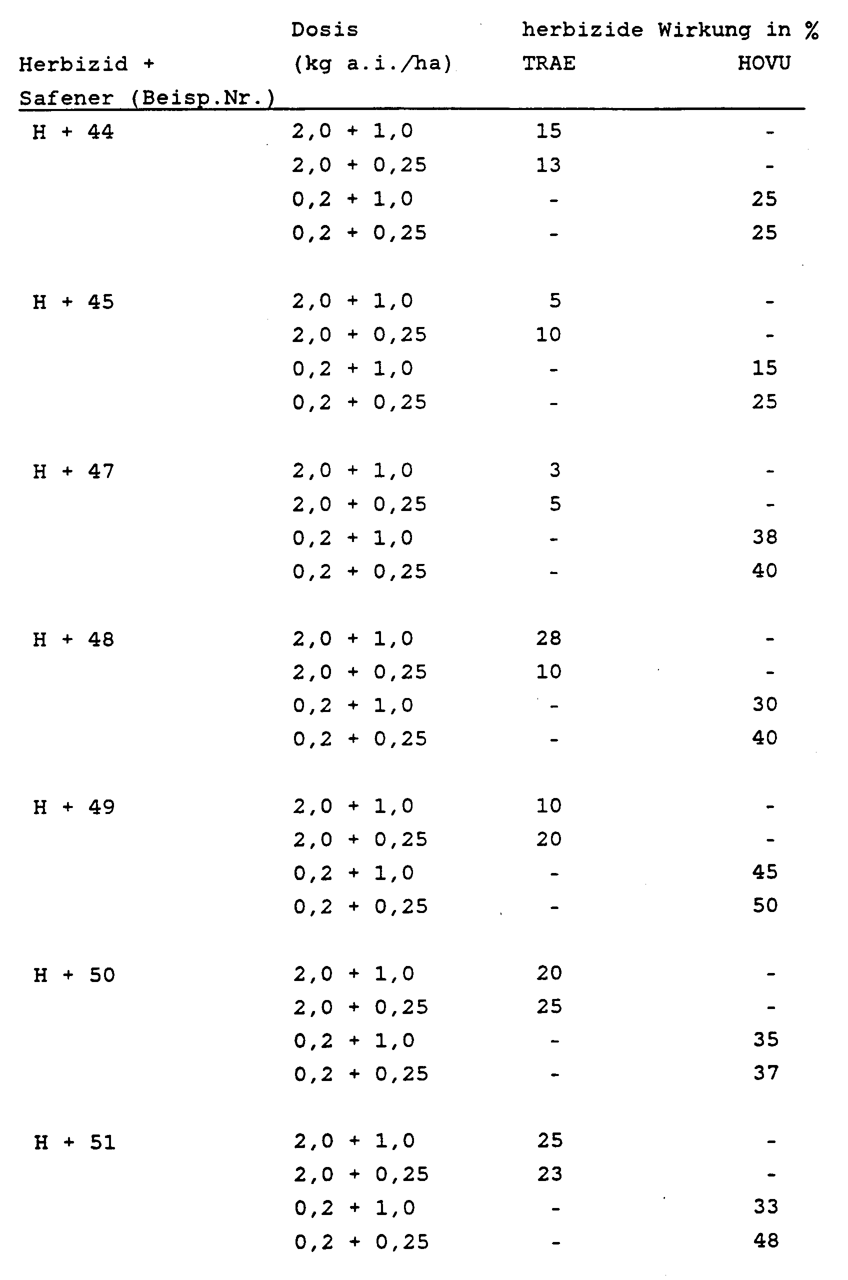

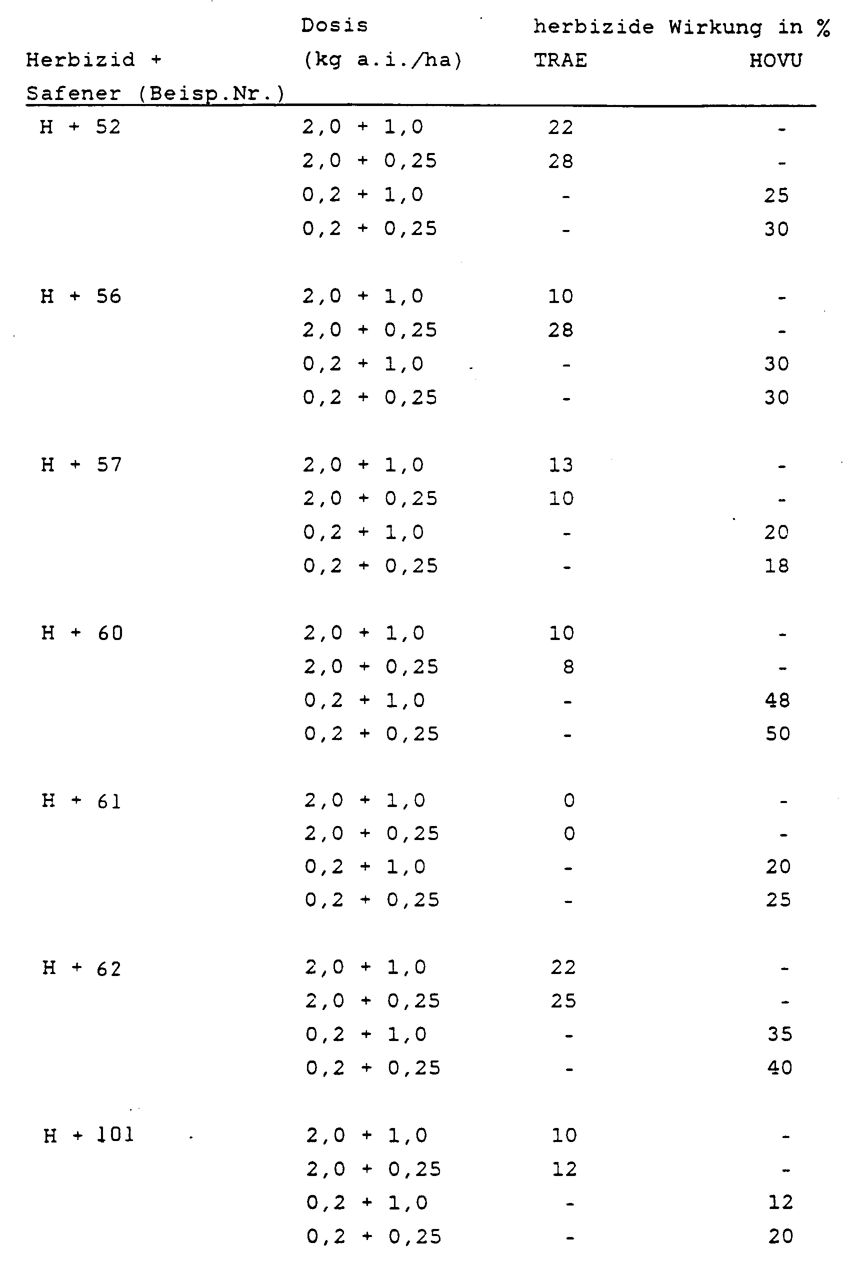

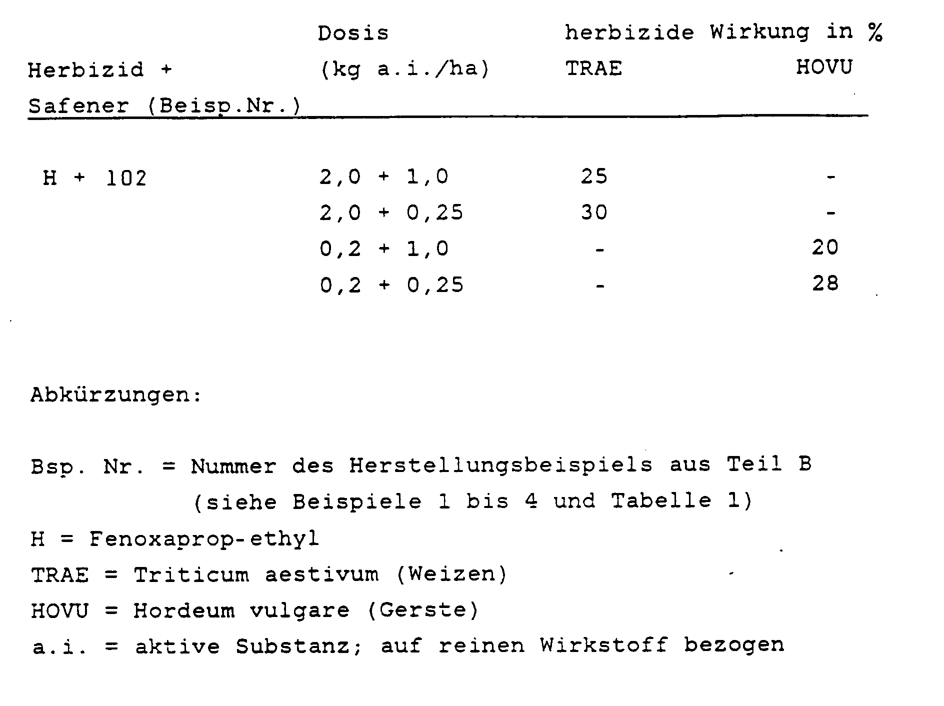

## Patentansprüche (Patentansprüche für folgende(n) Vertragsstaat(en): AT, BE, CH, LI, DE, DK, FR, GB, IT, NL, SE, GR)

1. Mittel für den Einsatz im Pflanzenschutz, dadurch gekennzeichnet, daß sie als Safener mindestens eine Verbindung der Formel (I) 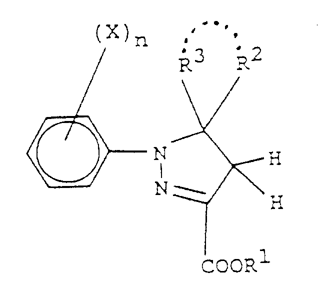 worin
X unabhängig voneinander Halogen oder Halogenalkyl,
n eine ganze Zahl von 1 bis 3,
R¹ Wasserstoff, Alkyl, Cycloalkyl, Trialkylsilyl, Trialkylsilylmethyl oder Alkyloxyalkyl,
R² und R³ unabhängig voneinander Wasserstoff, Alkyl, C₃-C₆-Cycloalkyl, Alkenyl, Alkinyl, Halogenalkyl, Alkoxyalkyl, Hydroxyalkyl, Alkoxycarbonyl, Alkylcarbonyl, Alkylaminocarbonyl, gegebenenfalls substituiertes Phenyl, Halogen oder Cyano bedeuten, wobei die Reste R² und R³ mit dem 5-C-Atom des Pyrazolinrings einen Ring bilden können,
und mindestens ein Herbizid enthalten.

2. Mittel nach Anspruch 1, dadurch gekennzeichnet, daß
X unabhängig voneinander Halogen oder C₁-C₄-Halogenalkyl,
n eine ganze Zahl von 1 bis 3,
R¹ C₁-C₆-Alkyl, C₃-C₆-Cycloalkyl, Tri-(C₁-C₄-alkyl)-silyl, Tri(C₁-C₄-alkyl)-silylmethyl oder (C₁-C₆-Alkyloxy)-C₁-C₆-alkyl,
R² und R³ unabhängig voneinander Wasserstoff, C₁-C₆-Alkyl, C₂-C₆-Alkenyl, C₂-C₆-Alkinyl, C₃-C₆-Cycloalkyl, C₁-C₆-Halogenalkyl, Mono- oder Di-(C₁-C₄-Alkoxy)-C₁-C₄-alkyl, C₁-C₆-Hydroxyalkyl, (C₁-C₆-Alkyl)-carbonyl, Mono- oder Di-(C₁-C₄-Alkyl)-aminocarbonyl, Cyano, Halogen, (C₁-C₁₂-Alkoxy)-carbonyl, Phenyl oder Phenyl, das durch einen oder mehrere Reste aus der Gruppe Halogen, C₁-C₄-Alkyl, C₁-C₄-Alkoxy und Cyano substituiert ist, bedeuten.

3. Mittel nach Anspruch 1 oder 2, dadurch gekennzeichnet, daß
X unabhängig voneinander Fluor, Chlor, Brom oder Trifluormethyl,
n 2 oder 3,
R¹ C₁-C₄-Alkyl oder (C₁-C₄-Alkyloxy)-C₁-C₄-alkyl,
R² Wasserstoff, C₁-C₄-Alkyl oder C₂-C₄-Alkenyl,
R³ Wasserstoff, C₁-C₄-Alkyl, C₃-C₄-Alkenyl, C₂-C₄-Alkinyl, C₁-C₄-Halogenalkyl, Mono- oder Di-(C₁-C₄-Alkoxy)-C₁-C₄-alkyl, C₁-C₄-Hydroxyalkyl, Mono- oder Di-(C₁-C₄-Alkyl)-aminocarbonyl, Cyano, (C₁-C₁₂-Alkoxy)-carbonyl, Phenyl oder Phenyl, das durch einen oder mehrere Reste aus der Gruppe Halogen substituiert ist, bedeuten.

4. Mittel nach Anspruch 1, 2 oder 3, dadurch gekennzeichnet, daß
X unabhängig voneinander Fluor, Chlor, Brom oder Trifluormethyl,
n 2 oder 3,
R² Wasserstoff, C₁-C₄-Alkyl oder C₂-C₄-Alkenyl,
R³ C₁-C₄-Alkyl, das unsubstituiert oder ein- oder mehrfach durch Halogen substituiert ist, oder (C₁-C₁₂-Alkoxy)-carbonyl oder Cyano bedeuten.

5. Mittel nach einem oder mehreren der Ansprüche 1 bis 4, dadurch gekennzeichnet, daß sie 1 bis 95 Gew.-% der Kombination aus Verbindung der Formel (I) und Herbizid und 99 bis 5 Gew.-% an im Pflanzenschutz üblichen Formulierungshilfsmitteln enthalten.

6. Mittel zum Schutz vor phytotoxischen Nebenwirkungen von Herbiziden an Kulturpflanzen, dadurch gekennzeichnet, daß sie mindestens eine Verbindung der Formel (I), wie sie in einem oder mehreren der Ansprüche 1 bis 4 definiert ist, und mindestens ein Tensid oder einen Füll- oder Trägerstoff und gegebenenfalls andere im Pflanzenschutz übliche Formulierungshilfsmittel enthalten.

7. Verbindung der in einem oder mehreren der Ansprüche 1 bis 4 definierten Formel (I), dadurch gekennzeichnet, daß
R¹ Cycloalkyl, Trialkylsilyl, Trimethylsilylmethyl oder Alkoxyalkyl bedeutet und
X, n, R² und R³ die in Formel (I) definierte Bedeutung haben oder daß
n 2 oder 3 bedeutet und
X, R¹, R² und R³ die in Formel (I) definierte Bedeutung haben.

8. Verbindung nach Anspruch 7, dadurch gekennzeichnet, daß
(X)ₙ zwei Reste aus den Gruppen Halogen und C₁-C₄-Halogenalkyl bedeuten.

9. Verbindung nach Anspruch 8, dadurch gekennzeichnet, daß
(X)ₙ 2,4-Cl₂, 2,4-F₂, 2,4-Br₂, 2-Cl-4-F, 2-F-4-Cl, 2,4-(CF₃)₂, 2-CF₃-4-Cl, 2-Cl-4-CF₃, 2-F-4-CF₃, 2-CF₃-4-F, 2-CF₃-4-Br oder 2-Br-4-CF₃ bedeuten.

10. Verbindung nach Anspruch 7, dadurch gekennzeichnet, daß
(X)ₙ 2,4-Cl₂,
R¹ H oder C₁-C₄-Alkyl,
R² H oder C₁-C₄-Alkyl und
R³ C₁-C₄-Alkyl oder (C₁-C₁₂-Alkoxy)-carbonyl bedeuten.

11. Verbindung nach Anspruch 10, dadurch gekennzeichnet, daß
(X)ₙ 2,4-Dichlor,
R¹ Ethyl,
R² Methyl und
R³ Ethoxy-carbonyl bedeuten.

12. Verfahren zur Herstellung einer nach einem der Ansprüche 7 bis 11 definierten Verbindung der Formel (I), dadurch gekennzeichnet, daß man eine Verbindung der Formel (II), 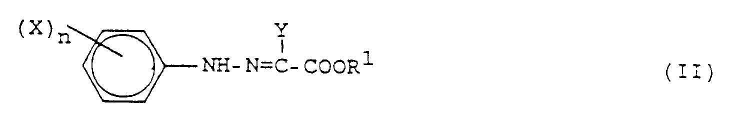 worin Y für Chlor oder Brom steht und (X)ₙ und R¹ die in Formel (I) definierte Bedeutung haben, mit einem Olefin der Formel (III), 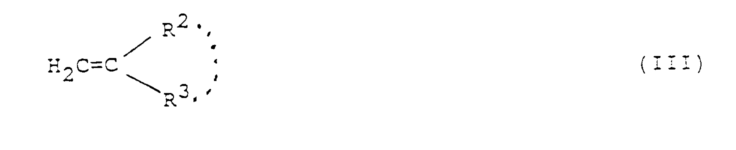 worin R² und R³ wie in Formel (I) definiert sind, umsetzt.

13. Verfahren zur selektiven Bekämpfung von unerwünschten Pflanzen in Nutzpflanzenkulturen, dadurch gekennzeichnet, daß man ein oder mehrere Herbizide in Kombination mit einem oder mehreren Safenern aus der Gruppe der nach einem der Ansprüche 1 bis 4 und 7 bis 11 definierten Verbindungen der Formel (I) auf die Pflanzen, Pflanzensamen oder die Anbaufläche appliziert.

14. Verwendung von nach einem der Ansprüche 1 bis 4 und 7 bis 11 definierten Verbindungen der Formel (I) zum Schutz von Nutzpflanzen vor phytotoxischen Nebenwirkungen von Herbiziden.

## Patentansprüche (Patentansprüche für folgende(n) Vertragsstaat(en): ES)

1. Mittel für den Einsatz im Pflanzenschutz, dadurch gekennzeichnet, daß sie als Safener mindestens eine Verbindung der Formel (I) 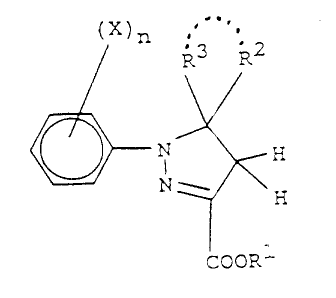 worin
X unabhängig voneinander Halogen oder Halogenalkyl,
n eine ganze Zahl von 1 bis 3,
R¹ Wasserstoff, Alkyl, Cycloalkyl, Trialkylsilyl, Trialkylsilylmethyl oder Alkyloxyalkyl,
R² und R³ unabhängig voneinander Wasserstoff, Alkyl, C₃-C₆-Cycloalkyl, Alkenyl, Alkinyl, Halogenalkyl, Alkoxyalkyl, Hydroxyalkyl, Alkoxycarbonyl, Alkylcarbonyl, Alkylaminocarbonyl, gegebenenfalls substituiertes Phenyl, Halogen oder Cyano bedeuten, wobei die Reste R² und R³ mit dem 5-C-Atom des Pyrazolinrings einen Ring bilden können,
und mindestens ein Herbizid enthalten.

2. Mittel nach Anspruch 1, dadurch gekennzeichnet, daß
X unabhängig voneinander Halogen oder C₁-C₄-Halogenalkyl,
n eine ganze Zahl von 1 bis 3,
R¹ C₁-C₆-Alkyl, C₃-C₆-Cycloalkyl, Tri-(C₁-C₄-alkyl)-silyl, Tri(C₁-C₄-alkyl)-silylmethyl oder (C₁-C₆-Alkyloxy)-C₁-C₆-alkyl,
R² und R³ unabhängig voneinander Wasserstoff, C₁-C₆-Alkyl, C₂-C₆-Alkenyl, C₂-C₆-Alkinyl, C₃-C₆-Cycloalkyl, C₁-C₆-Halogenalkyl, Mono- oder Di-(C₁-C₄-Alkoxy)-C₁-C₄-alkyl, C₁-C₆-Hydroxyalkyl, (C₁-C₆-Alkyl)-carbonyl, Mono- oder Di-(C₁-C₄-Alkyl)-aminocarbonyl, Cyano, Halogen, (C₁-C₁₂-Alkoxy)-carbonyl, Phenyl oder Phenyl, das durch einen oder mehrere Reste aus der Gruppe Halogen, C₁-C₄-Alkyl, C₁-C₄-Alkoxy und Cyano substituiert ist, bedeuten.

3. Mittel nach Anspruch 1 oder 2, dadurch gekennzeichnet, daß
X unabhängig voneinander Fluor, Chlor, Brom oder Trifluormethyl,
n 2 oder 3,
R¹ C₁-C₄-Alkyl oder (C₁-C₄-Alkyloxy)-C₁-C₄-alkyl,
R² Wasserstoff, C₁-C₄-Alkyl oder C₂-C₄-Alkenyl,
R³ Wasserstoff, C₁-C₄-Alkyl, C₃-C₄-Alkenyl, C₂-C₄-Alkinyl, C₁-C₄-Halogenalkyl, Mono- oder Di-(C₁-C₄-Alkoxy)-C₁-C₄-alkyl, C₁-C₄-Hydroxyalkyl, Mono- oder Di-(C₁-C₄-Alkyl)-aminocarbonyl, Cyano, (C₁-C₁₂-Alkoxy)-carbonyl, Phenyl oder Phenyl, das durch einen oder mehrere Reste aus der Gruppe Halogen substituiert ist, bedeuten.

4. Mittel nach Anspruch 1, 2 oder 3, dadurch gekennzeichnet, daß
X unabhängig voneinander Fluor, Chlor, Brom oder Trifluormethyl,
n 2 oder 3,
R² Wasserstoff, C₁-C₄-Alkyl oder C₂-C₄-Alkenyl,
R³ C₁-C₄-Alkyl, das unsubstituiert oder ein- oder mehrfach durch Halogen substituiert ist, oder (C₁-C₁₂-Alkoxy)-carbonyl oder Cyano bedeuten.

5. Mittel nach einem oder mehreren der Ansprüche 1 bis 4, dadurch gekennzeichnet, daß sie 1 bis 95 Gew.-% der Kombination aus Verbindung der Formel (I) und Herbizid und 99 bis 5 Gew.-% an im Pflanzenschutz üblichen Formulierungshilfsmitteln enthalten.

6. Mittel zum Schutz vor phytotoxischen Nebenwirkungen von Herbiziden an Kulturpflanzen, dadurch gekennzeichnet, daß sie mindestens eine Verbindung der Formel (I), wie sie in einem oder mehreren der Ansprüche 1 bis 4 definiert ist, und mindestens ein Tensid oder einen Füll- oder Trägerstoff und gegebenenfalls andere im Pflanzenschutz übliche Formulierungshilfsmittel enthalten.

7. Verfahren zur Herstellung einer Verbindung der in einem oder mehreren der Ansprüche 1 bis 4 definierten Formel (I), dadurch gekennzeichnet, daß
R¹ Cycloalkyl, Trialkylsilyl, Trimethylsilylmethyl oder Alkoxyalkyl bedeutet und
X, n, R² und R³ die in Formel (I) definierte Bedeutung haben oder daß
n 2 oder 3 bedeutet und
X, R¹, R² und R³ die in Formel (I) definierte Bedeutung haben,
dadurch gekennzeichnet, daß man eine Verbindung der Formel (II), 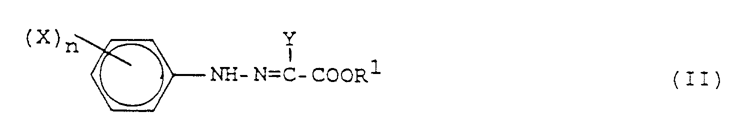 worin Y für Chlor oder Brom steht und (X)ₙ und R¹ die in Formel (I) definierte Bedeutung haben, mit einem Olefin der Formel (III), 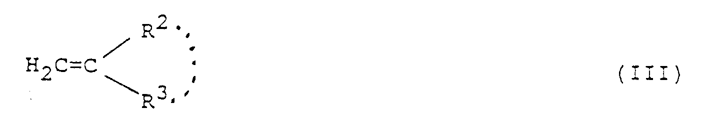 worin R² und R³ wie in Formel (I) definiert sind, umsetzt

8. Verfahren nach Anspruch 7, dadurch gekennzeichnet, daß
(X)ₙ zwei Reste aus den Gruppen Halogen und C₁-C₄-Halogenalkyl bedeuten.

9. Verfahren nach Anspruch 8, dadurch gekennzeichnet, daß
(X)ₙ 2,4-Cl₂, 2,4-F₂, 2,4-Br₂, 2-Cl-4-F, 2-F-4-Cl, 2,4-(CF₃)₂, 2-CF₃-4-Cl, 2-Cl-4-CF₃, 2-F-4-CF₃, 2-CF₃-4-F, 2-CF₃-4-Br oder 2-Br-4-CF₃ bedeuten.

10. Verfahren nach Anspruch 7, dadurch gekennzeichnet, daß
(X)ₙ 2,4-Cl₂,
R¹ H oder C₁-C₄-Alkyl,
R² H oder C₁-C₄-Alkyl und
R³ C₁-C₄-Alkyl oder (C₁-C₁₂-Alkoxy)-carbonyl bedeuten.

11. Verfahren nach Anspruch 10, dadurch gekennzeichnet, daß
(X)ₙ 2,4-Dichlor,
R¹ Ethyl,
R² Methyl und
R³ Ethoxy-carbonyl bedeuten.

12. Verfahren zur selektiven Bekämpfung von unerwünschten Pflanzen in Nutzpflanzenkulturen, dadurch gekennzeichnet, daß man ein oder mehrere Herbizide in Kombination mit einem oder mehreren Safenern aus der Gruppe der nach einem der Ansprüche 1 bis 4 und 7 bis 11 definierten Verbindungen der Formel (I) auf die Pflanzen, Pflanzensamen oder die Anbaufläche appliziert.

13. Verwendung von nach einem der Ansprüche 1 bis 4 und 7 bis 11 definierten Verbindungen der Formel (I) zum Schutz von Nutzpflanzen vor phytotoxischen Nebenwirkungen von Herbiziden.

## Claims (Claims for the following Contracting State(s): AT, BE, CH, LI, DE, DK, FR, GB, IT, NL, SE, GR)

1. A composition for use in plant protection, which contains, as safener, at least one compound of the formula (I) 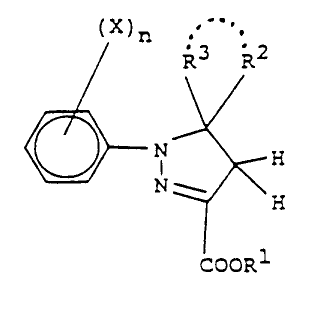 where
X radicals independently of one another are halogen or haloalkyl,
n is an integer from 1 to 3,
R¹ is hydrogen, alkyl, cycloalkyl, trialkylsilyl, trialkylsilylmethyl or alkyloxyalkyl,
R² and R³ independently of one another are hydrogen, alkyl, C₃-C₆-cycloalkyl, alkenyl, alkynyl, haloalkyl, alkoxyalkyl, hydroxyalkyl, alkoxycarbonyl, alkylcarbonyl, alkylaminocarbonyl, optionally substituted phenyl, halogen or cyano, it being possible for the radicals R² and R³ to form a ring with the 5-C atom of the pyrazoline ring,
and at least one herbicide.

2. A composition as claimed in claim 1, wherein
X radicals independently of one another are halogen or C₁-C₄-haloalkyl,
n is an integer from 1 to 3,
R¹ is C₁-C₆-alkyl, C₃-C₆-cycloalkyl, tri(C₁-C₄-alkyl)silyl, tri(C₁-C₄-alkyl)silylmethyl or (C₁-C₆-alkyloxy)-C₁-C₆-alkyl,
R² and R³ independently of one another are hydrogen, C₁-C₆-alkyl, C₂-C₆-alkenyl, C₂-C₆-alkynyl, C₃-C₆-cycloalkyl, C₁-C₆-haloalkyl, mono- or di(C₁-C₄-alkoxy)-C₁-C₄-alkyl, C₁-C₆-hydroxyalkyl, (C₁-C₆-alkyl)carbonyl, mono- or di(C₁-C₄-alkyl)aminocarbonyl, cyano, halogen, (C₁-C₁₂-alkoxy)carbonyl, phenyl, or phenyl which is substituted by one or more radicals selected from the group comprising halogen, C₁-C₄-alkyl, C₁-C₄-alkoxy and cyano.

3. A composition as claimed in claim 1 or 2, wherein
X radicals independently of one another are fluorine, chlorine, bromine or trifluoromethyl,
n is 2 or 3,
R¹ is C₁-C₄-alkyl or (C₁-C₄-alkyloxy)-C₁-C₄-alkyl,
R² is hydrogen, C₁-C₄-alkyl or C₂-C₄-alkenyl,
R³ is hydrogen, C₁-C₄-alkyl, C₃-C₄-alkenyl, C₂-C₄-alkynyl, C₁-C₄-haloalkyl, mono- or di(C₁-C₄-alkoxy)-C₁-C₄-alkyl, C₁-C₄-hydroxyalkyl, mono- or di(C₁-C₄-alkyl)-aminocarbonyl, cyano, (C₁-C₁₂-alkyloxy)carbonyl, phenyl, or phenyl which is substituted by one or more radicals selected from the group consisting of halogen.

4. An agent as claimed in claim 1, 2 or 3, wherein
X radicals independently of one another are fluorine, chlorine, bromine or trifluoromethyl,
n is 2 or 3,
R² is hydrogen, C₁-C₄-alkyl or C₂-C₄-alkenyl,
R³ is C₁-C₄-alkyl which is unsubstituted or monosubstituted or polysubstituted by halogen, or is (C₁-C₁₂-alkoxy)carbonyl or cyano.

5. An agent as claimed in one or more of claims 1 to 4, which contains 1 to 95% by weight of the combination of compound of the formula (I) and herbicide and 99 to 5% by weight of formulation auxiliaries which are customary in plant protection.

6. A composition for protection against phytotoxic secondary effects of herbicides on crop plants, which contains at least one compound of the formula (I) as defined in one or more of claims 1 to 4 and at least one surfactant or a filler or carrier and, if appropriate, other formulation auxiliaries which are customary in plant protection.

7. A compound of the formula (I) defined in one or more of claims 1 to 4, wherein
R¹ is cycloalkyl, trialkylsilyl, trimethylsilylmethyl or alkoxyalkyl and
X, n, R² and R³ have the meaning defined in formula (I), or wherein
n is 2 or 3 and
X, R¹, R² and R³ have the meaning defined in formula (I).

8. A compound as claimed in claim 7, wherein
(X)ₙ is two radicals selected from the groups comprising halogen and C₁-C₄-haloalkyl.

9. A compound as claimed in claim 8, wherein
(X)ₙ is 2,4-Cl₂, 2,4-F₂, 2,4-Br₂, 2-Cl-4-F, 2-F-4-Cl, 2,4-(CF₃)₂, 2-CF₃-4-Cl, 2-Cl-4-CF₃, 2-F-4-CF₃, 2-CF₃-4-F, 2-CF₃-4-Br or 2-Br-4-CF₃.

10. A compound as claimed in claim 7, wherein
(X)ₙ is 2,4-Cl₂,
R¹ is H or C₁-C₄-alkyl,
R² is H or C₁-C₄-alkyl, and
R³ is C₁-C₄-alkyl or (C₁-C₁₂-alkoxy)carbonyl.

11. A compound as claimed in claim 10, wherein
(X)ₙ is 2,4-dichloro,
R¹ is ethyl,
R² is methyl, and
R³ is ethoxycarbonyl

12. A process for preparing a compound of the formula (I) as defined in one of claims 7 to 11, which comprises reacting a compound of the formula (II) 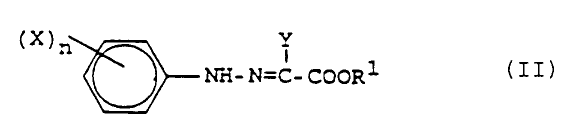 where Y is chlorine or bromine and (X)ₙ and R¹ have the meaning defined in formula (I), with an olefin of the formula (III) 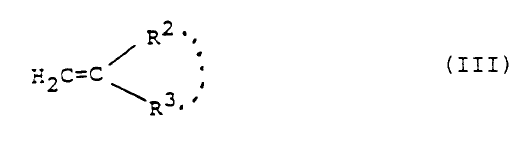 where R² and R³ are as defined in formula (I).

13. A method of selectively controlling undesired plants in crops of useful plants, which comprises applying one or more herbicides in combination with one or more safeners selected from the group consisting of the compounds of the formula (I) as defined in one of claims 1 to 4 and 7 to 11, to the plants, seeds of the plants, or the area under cultivation.

14. The use of a compound of the formula (I) as defined in one of claims 1 to 4 and 7 to 11 for protection of useful plants against phytotoxic secondary effects of herbicides.

## Claims (Claims for the following Contracting State(s): ES)

1. An agent for use in plant protection, which contains, as safener, at least one compound of the formula (I) 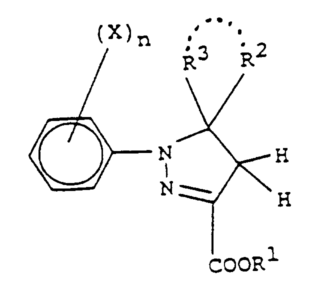 where
X radicals independently of one another are halogen or haloalkyl,
n is an integer from 1 to 3,
R¹ is hydrogen, alkyl, cycloalkyl, trialkylsilyl, trialkylsilylmethyl or alkyloxyalkyl,
R² and R³ independently of one another are hydrogen, alkyl, C₃-C₆-cycloalkyl, alkenyl, alkynyl, haloalkyl, alkoxyalkyl, hydroxyalkyl, alkoxycarbonyl, alkylcarbonyl, alkylaminocarbonyl, optionally substituted phenyl, halogen or cyano, it being possible for the radicals R² and R³ to form a ring with the 5-C atom of the pyrazoline ring,
and at least one herbicide.

2. An agent as claimed in claim 1, wherein
X radicals independently of one another are halogen or C₁-C₄-haloalkyl,
n is an integer from 1 to 3,
R¹ is C₁-C₆-alkyl, C₃-C₆-cycloalkyl, tri(C₁-C₄-alkyl)silyl, tri(C₁-C₄-alkyl)silylmethyl or (C₁-C₆-alkyloxy)-C₁-C₆-alkyl,
R² and R³ independently of one another are hydrogen, C₁-C₆-alkyl, C₂-C₆-alkenyl, C₂-C₆-alkynyl, C₃-C₆-cycloalkyl, C₁-C₆-haloalkyl, mono- or di(C₁-C₄-alkoxy)-C₁-C₄-alkyl, C₁-C₆-hydroxyalkyl, (C₁-C₆-alkyl)carbonyl, mono- or di(C₁-C₄-alkyl)aminocarbonyl, cyano, halogen, (C₁-C₁₂-alkoxy)carbonyl, phenyl, or phenyl which is substituted by one or more radicals selected from the group comprising halogen, C₁-C₄-alkyl, C₁-C₄-alkoxy or cyano.

3. An agent as claimed in claim 1 or 2, wherein
X radicals independently of one another are fluorine, chlorine, bromine or trifluoromethyl,
n is 2 or 3,
R¹ is C₁-C₄-alkyl or (C₁-C₄-alkyloxy)-C₁-C₄-alkyl,
R² is hydrogen, C₁-C₄-alkyl or C₂-C₄-alkenyl,
R³ is hydrogen C₁-C₄-alkyl, C₃-C₄-alkenyl, C₂-C₄-alkynyl, C₁-C₄-haloalkyl, mono- or di(C₁-C₄-alkoxy)-C₁-C₄-alkyl, C₁-C₄-hydroxyalkyl, mono- or di(C₁-C₄-alkyl)-aminocarbonyl, cyano, (C₁-C₁₂-alkoxy)carbonyl, phenyl, or phenyl which is substituted by one or more radicals selected from the group consisting of halogen.

4. An agent as claimed in claim 1, 2 or 3, wherein
X radicals independently of one another are fluorine, chlorine, bromine or trifluoromethyl,
n is 2 or 3,
R² is hydrogen, C₁-C₄-alkyl or C₂-C₄-alkenyl,
R³ is C₁-C₄-alkyl which is unsubstituted or monosubstituted or polysubstituted by halogen, or is (C₁-C₁₂-alkoxy)carbonyl or cyano.

5. An agent as claimed in one or more of claims 1 to 4, which contains 1 to 95% by weight of the combination of compound of the formula (I) and herbicide and 99 to 5% by weight of formulation auxiliaries which are customary in plant protection.

6. A composition for protection against phytotoxic secondary effects of herbicides on crop plants, which contains at least one compound of the formula (I) as defined in one or more of claims 1 to 4 and at least one surfactant or a filler or carrier and, if appropriate, other formulation auxiliaries which are customary in plant protection.

7. A process for preparing a compound of the formula (I) defined in one or more of claims 1 to 4, in which
R¹ is cycloalkyl, trialkylsilyl, trimethylsilylmethyl or alkoxyalkyl and
X, n, R² and R³ have the meaning defined in formula (I), or
n is 2 or 3 and
X, R¹, R² and R³ have the meaning defined in formula (I),
which comprises reacting a compound of the formula (II) 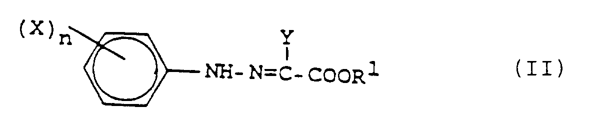 where Y is chlorine or bromine and (X)ₙ and R¹ have the meaning defined in formula (I), with an olefin of the formula (III) 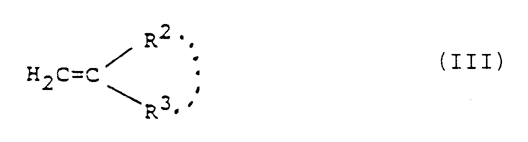 where R² and R³ are as defined in formula (I).

8. The process as claimed in claim 7, wherein
(X)ₙ is two radicals selected from the groups comprising halogen and C₁-C₄-haloalkyl.

9. The process as claimed in claim 8, wherein
(X)ₙ is 2,4-Cl₂, 2,4-F₂, 2,4-Br₂, 2-Cl-4-F, 2-F-4-Cl, 2,4-(CF₃)₂, 2-CF₃-4-Cl, 2-Cl-4-CF₃, 2-F-4-CF₃, 2-CF₃-4-F, 2-CF₃-4-Br or 2-Br-4-CF₃.

10. The process as claimed in claim 7, wherein
(X)ₙ is 2,4-Cl₂,
R¹ is H or C₁-C₄-alkyl,
R² is H or C₁-C₄-alkyl, and
R³ is C₁-C₄-alkyl or (C₁-C₁₂-alkoxy)carbonyl.

11. The process as claimed in claim 10, whereine
(X)ₙ is 2,4-dichloro,
R¹ is ethyl,
R² is methyl, and
R³ is ethoxycarbonyl.

12. A method of selectively controlling undesired plants in crops of useful plants, which comprises applying one or more herbicides in combination with one or more safeners selected from the group consisting of the compounds of the formula (I) as defined in one of claims 1 to 4 and 7 to 11, to the plants, seeds of the plants, or the area under cultivation.

13. The use of a compound of the formula (I) as defined in one of claims 1 to 4 and 7 to 11 for protection of useful plants against phytotoxic secondary effects of herbicides.

## Revendications (Revendications pour l'(les) Etat(s) contractant(s) suivant(s): AT, BE, CH, LI, DE, DK, FR, GB, IT, NL, SE, GR)

1. Agents pour utilisation en protection des végétaux, caractérisés en ce qu'ils contiennent en tant qu'antidote au moins un composé de formule (I) : 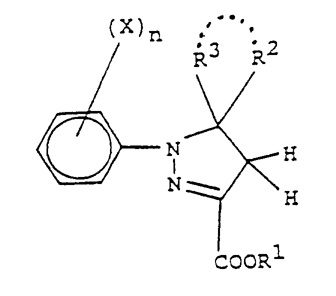 dans laquelle :
les radicaux X, indépendamment les uns des autres, sont chacun un atome d'halogène ou un groupe halogénalkyle,
n est un nombre entier de 1 à 3,
R¹ est un atome d'hydrogène ou un groupe alkyle, cycloalkyle, trialkylsilyle, trialkylsilylméthyle ou alkyloxyalkyle,
R² et R³, indépendamment l'un de l'autre, sont chacun un atome d'hydrogène, un groupe alkyle, cycloalkyle en C₃-C₆, alcényle, alcynyle, halogénalkyle, alcoxyalkyle, hydroxyalkyle, alcoxycarbonyle, alkylcarbonyle, alkylaminocarbonyle, phényle éventuellement substitué, halogéno ou cyano, les radicaux R² et R³ pouvant, avec l'atome de carbone en position 5 du noyau pyrazoline, former un radical cyclique,
et au moins un herbicide.

2. Agents selon la revendication 1, caractérisés en ce que :
les groupes X, indépendamment les uns des autres, sont chacun un atome d'halogène ou un groupe halogénalkyle en C₁-C₄,
n est un nombre entier de 1 à 3,
R¹ est un groupe alkyle en C₁-C₆, cycloalkyle en C₃-C₆, tri(alkyle en C₁-C₄)-silyle, tri(alkyle en C₁-C₄)-silylméthyle ou (alkyloxy en C₁-C₆)-(alkyle en C₁-C₆),
R² et R³, indépendamment l'un de l'autre, sont chacun un atome d'hydrogène, un groupe alkyle en C₁-C₆, alcényle en C₂-C₆, alcynyle en C₂-C₆, cycloalkyle en C₃-C₆, halogénalkyle en C₁-C₆, mono- ou di(alcoxy en C₁-C₄)-(alkyle en C₁-C₄), hydroxyalkyle en C₁-C₆, (alkyle en C₁-C₆)carbonyle, mono- ou di-(alkyle en C₁-C₄)aminocarbonyle, cyano, halogéno, (alkyle en C₁-C₁₂)-oxycarbonyle, phényle, ou encore phényle substitué par un ou plusieurs substituants du groupe comprenant les halogènes et les groupes alkyle en C₁-C₄, alcoxy en C₁-C₄ et cyano.

3. Agents selon la revendication 1 ou 2, caractérisés en ce que :
les groupes X sont chacun indépendamment des autres un groupe fluoro, chloro, bromo ou trifluorométhyle, chacun de préférence en position 2 ou 4,
n vaut 2 ou 3,
R¹ est un groupe alkyle en C₁-C₄ ou (alcoxy en C₁-C₄)-(alkyle en C₁-C₄),
R² est un hydrogène, un groupe alkyle en C₁-C₄, alcényle en C₂-C₄,
R³ est un groupe alkyle en C₁-C₄, alcényle en C₃-C₄, alcynyle en C₂-C₄, halogénalkyle en C₁-C₄, mono- ou di-(alcoxy en C₁-C₄)-(alkyle en C₁-C₄), hydroxyalkyle en C₁-C₄, mono- ou di-(alkyle en C₁-C₄)aminocarbonyle, cyano, (alkyloxy en C₁-C₁₂)-carbonyle, phényle, ou phényle une ou plusieurs fois substitué par des halogènes, en particulier le fluor ou le chlore.

4. Agents selon les revendications 1, 2 ou 3, caractérisés en ce que :
les groupes X, indépendamment les uns des autres, sont des groupes fluoro, chloro, bromo ou trifluorométhyle, de préférence en position 2 ou 4 du noyau phényle,
n vaut 2 ou 3,
R² est un atome d'hydrogène ou un groupe alkyle en C₁-C₄, alcényle en C₂-C₄, ou alcynyle en C₂-C₄,
R³ est un groupe alkyle en C₁-C₄, qui est non substitué ou une ou plusieurs fois substitué par des groupes halogéno, un groupe (alkyle en C₁-C₁₂)oxycarbonyle ou cyano.

5. Agents selon l'une ou plusieurs des revendications 1 à 4, caractérisés en ce qu'ils contiennent de 1 à 95 % en poids dc la combinaison du composé de formule (I) et d'un herbicide, et de 99 à 5 % en poids d'adjuvants de formulations usuelles en protection des végétaux.

6. Agents pour assurer une protection contre les effets secondaires phytotoxiques d'herbicides sur les plantes de culture, caractérisés en ce qu'ils contiennent au moins un composé de formule (I), tel que défini dans une ou plusieurs des revendications 1 à 4, et au moins un tensioactif ou une charge ou un support, et éventuellement d'autres adjuvants de formulations usuelles en protection des végétaux.

7. Composé ayant la formule (I) définie dans une ou plusieurs des revendications 1 à 4, caractérisé en ce que :
R¹ est un groupe cycloalkyle, trialkylsilyle, triméthylsilylméthyle ou alcoxyalkyle, et
X, n, R² et R³ ont les significations données à propos de la formule (I), ou bien que
n vaut 2 ou 3, et
X, R¹, R² et R³ ont les significations données à propos de la formule (I).

8. Composé selon la revendication 7, caractérisé en ce que (X)ₙ représente deux radicaux choisis parmi les halogènes et les groupes halogénalkyle en C₁-C₄.

9. Composé selon la revendication 8, caractérisé en ce que (X)ₙ est 2,4-Cl₂, 2,4-F₂, 2,4-Br₂, 2-Cl-4-F, 2-F-4-Cl, 2,4-(CF₃)₂, 2-CF₃-4-Cl, 2-Cl-4-CF₃, 2-F-4-CF₃, 2-CF₃-4-F, 2-CF₃-4-Br ou 2-Br-4-CF₃

10. Composé selon la revendication 7, caractérisé en ce que :
(X)ₙ est 2,4-Cl₂,
R¹ est H ou un groupe alkyle en C₁-C₄,
R² est H ou un groupe alkyle en C₁-C₄, et
R³ est un groupe alkyle en C₁-C₄ ou (alcoxy en C₁-C₁₂)-carbonyle.

11. Composé selon la revendication 10, caractérisé en ce que :
(X)ₙ est le groupe 2,4-dichloro,
R¹ est le groupe éthyle,
R² est le groupe méthyle, et
R³ est le groupe éthoxycarbonyle.

12. Procédé pour préparer un composé de formule (I) selon l'une des revendications 7 à 11, caractérisé en ce qu'on fait réagir un composé de formule (II) : 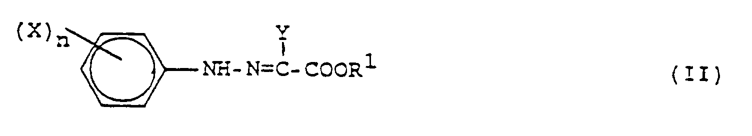 dans laquelle Y est un atome de chlore ou de brome et (X)ₙ et R¹ ont les significations données à propos de la formule (I), avec une oléfine de formule (III) : 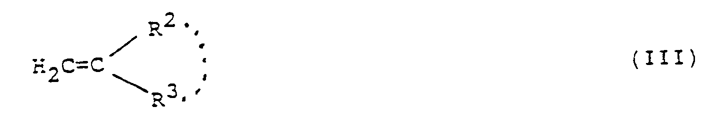 dans laquelle R² et R³ sont tels que définis dans la formule (I).

13. Procédé de maîtrise sélective des végétaux indésirables dans les cultures de plantes utiles, caractérisé en ce qu'on applique un ou plusieurs herbicides, en combinaison avec un ou plusieurs antidotes choisis dans le groupe des composés de formule (I) selon l'une des revendications 1 à 4 et 7 à 11, sur les plantes, semences végétales ou surfaces cultivées.

14. Utilisation de composés de formule (I) selon l'une des revendications 1 à 4 et 7 à 11, pour assurer une protection des plantes utiles contre les effets secondaires phytotoxiques des herbicides.

## Revendications (Revendications pour l'(les) Etat(s) contractant(s) suivant(s): ES)

1. Agents pour utilisation en protection des végétaux, caractérisés en ce qu'ils contiennent en tant qu'antidote au moins un composé de formule (I) : 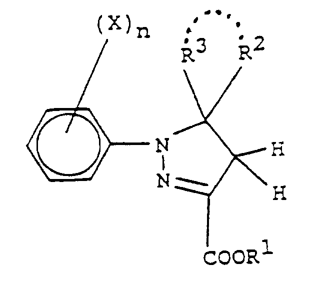 dans laquelle :
les radicaux X, indépendamment les uns des autres, sont chacun un atome d'halogène ou un groupe halogénalkyle,
n est un nombre entier de 1 à 3,
R¹ est un atome d'hydrogène ou un groupe alkyle, cycloalkyle, trialkylsilyle, trialkylsilylméthyle ou alkyloxyalkyle,
R² et R³, indépendamment l'un de l'autre, sont chacun un atome d'hydrogène, un groupe alkyle, cycloalkyle en C₃-C₆, alcényle, alcynyle, halogénalkyle, alcoxyalkyle, hydroxyalkyle, alcoxycarbonyle, alkylcarbonyle, alkylaminocarbonyle, phényle éventuellement substitué, halogéno ou cyano, les radicaux R² et R³ pouvant, avec l'atome de carbone en position 5 du noyau pyrazoline, former un radical cyclique,
et au moins un herbicide.

2. Agents selon la revendication 1, caractérisés en ce que :
les groupes X, indépendamment les uns des autres, sont chacun un atome d'halogène ou un groupe halogénalkyle en C₁-C₄,
n est un nombre entier de 1 à 3,
R¹ est un groupe alkyle en C₁-C₆, cycloalkyle en C₃-C₆, tri(alkyle en C₁-C₄)-silyle, tri(alkyle en C₁-C₄)-silylméthyle ou (alkyloxy en C₁-C₆)-(alkyle en C₁-C₆),
R² et R³, indépendamment l'un de l'autre, sont chacun un atome d'hydrogène, un groupe alkyle en C₁-C₆, alcényle en C₂-C₆, alcynyle en C₂-C₆, cycloalkyle en C₃-C₆, halogénalkyle en C₁-C₆, mono- ou di(alcoxy en C₁-C₄)-(alkyle en C₁-C₄), hydroxyalkyle en C₁-C₆, (alkyle en C₁-C₆)carbonyle, mono- ou di-(alkyle en C₁-C₄)aminocarbonyle, cyano, halogéno, (alkyle en C₁-C₁₂)-oxycarbonyle, phényle, ou encore phényle substitué par un ou plusieurs substituants du groupe comprenant les halogènes et les groupes alkyle en C₁-C₄, alcoxy en C₁-C₄ et cyano.

3. Agents selon la revendication 1 ou 2, caractérisés en ce que :
les groupes X sont chacun indépendamment des autres un groupe fluoro, chloro, bromo ou trifluorométhyle, chacun de préférence en position 2 ou 4,
n vaut 2 ou 3,
R¹ est un groupe alkyle en C₁-C₄ ou (alcoxy en C₁-C₄)-(alkyle en C₁-C₄),
R² est un hydrogène, un groupe alkyle en C₁-C₄, alcényle en C₂-C₄,
R³ est un groupe alkyle en C₁-C₄, alcényle en C₃-C₄, alcynyle en C₂-C₄, halogénalkyle en C₁-C₄, mono- ou di-(alcoxy en C₁-C₄)-(alkyle en C₁-C₄), hydroxyalkyle en C₁-C₄, mono- ou di-(alkyle en C₁-C₄)aminocarbonyle, cyano, (alkyloxy en C₁-C₁₂)-carbonyle, phényle, ou phényle une ou plusieurs fois substitué par des halogènes, en particulier le fluor ou le chlore.

4. Agents selon les revendications 1, 2 ou 3, caractérisés en ce que :
les groupes X, indépendamment les uns des autres, sont des groupes fluoro, chloro, bromo ou trifluorométhyle, de préférence en position 2 ou 4 du noyau phényle,
n vaut 2 ou 3,
R² est un atome d'hydrogène ou un groupe alkyle en C₁-C₄, alcényle en C₂-C₄, ou alcynyle en C₂-C₄,
R³ est un groupe alkyle en C₁-C₄, qui est non substitué ou une ou plusieurs fois substitué par des groupes halogéno, un groupe (alkyle en C₁-C₁₂)oxycarbonyle ou cyano.

5. Agents selon l'une ou plusieurs des revendications 1 à 4, caractérisés en ce qu'ils contiennent de 1 à 95 % en poids de la combinaison du composé de formule (I) et d'un herbicide, et de 99 à 5 % en poids d'adjuvants de formulations usuelles en protection des végétaux.

6. Agents pour assurer une protection contre les effets secondaires phytotoxiques d'herbicides sur les plantes de culture, caractérisés en ce qu'ils contiennent au moins un composé de formule (I), tel que défini dans une ou plusieurs des revendications 1 à 4, et au moins un tensioactif ou une charge ou un support, et éventuellement d'autres adjuvants de formulations usuelles en protection des végétaux.

7. Procédé pour préparer un composé ayant la formule (I) selon l'une ou plusieurs des revendications 1 à 4, dans lequel :
R¹ est un groupe cycloalkyle, trialkylsilyle, triméthylsilylméthyle ou alcoxyalkyle, et
X, n, R² et R³ ont les significations données à propos de la formule (I), ou bien que
n vaut 2 ou 3, et
X, R¹, R² et R³ ont les significations données à propos de la formule (I),
caractérisé en ce qu'on fait réagir un composé de formule (II) : 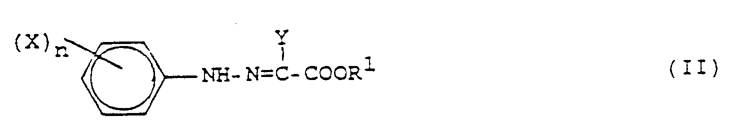 dans laquelle Y est un atome de chlore ou de brome et (X)ₙ et R¹ ont les significations données à propos de la formule (I), avec une oléfine de formule (III) : 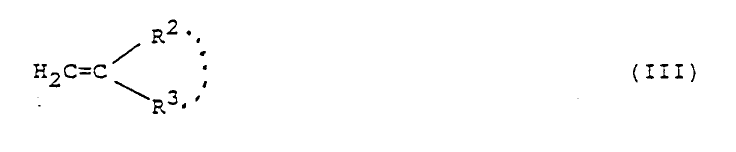 dans laquelle R² et R³ sont tels que définis dans la formule (I).

8. Procédé selon la revendication 7, caractérisé en ce que (X)ₙ représente deux radicaux choisis parmi les halogènes et les groupes halogénalkyle en C₁-C₄.

9. Procédé selon la revendication 8, caractérisé en ce que (X)ₙ est 2,4-Cl₂, 2,4-F₂, 2,4-Br₂, 2-Cl-4-F, 2-F-4-Cl, 2,4-(CF₃)₂, 2-CF₃-4-Cl, 2-Cl-4-CF₃, 2-F-4-CF₃, 2-CF₃-4-F, 2-CF₃-4-Br ou 2-Br-4-CF₃

10. Procédé selon la revendication 7, caractérisé en ce que :
(X)ₙ est 2,4-Cl₂,
R¹ est H ou un groupe alkyle en C₁-C₄,
R² est H ou un groupe alkyle en C₁-C₄, et
R³ est un groupe alkyle en C₁-C₄ ou (alcoxy en C₁-C₁₂)-carbonyle.

11. Procédé selon la revendication 10, caractérisé en ce que :
(X)ₙ est le groupe 2,4-dichloro,
R¹ est le groupe éthyle,
R² est le groupe méthyle, et
R³ est le groupe éthoxycarbonyle.

12. Procédé de maîtrise sélective des végétaux indésirables dans les cultures de plantes utiles, caractérisé en ce qu'on applique un ou plusieurs herbicides, en combinaison avec un ou plusieurs antidotes choisis dans le groupe des composés de formule (I) selon l'une des revendications 1 à 4 et 7 à 11, sur les plantes, semences végétales ou surfaces cultivées.

13. Utilisation de composés de formule (I) selon l'une des revendications 1 à 4 et 7 à 11, pour assurer une protection des plantes utiles contre les effets secondaires phytotoxiques des herbicides.
